(19) Europäisches Patentamt
European Patent Office
Office européen des brevets

(11) **EP 2 122 523 B1**

(12) **FASCICULE DE BREVET EUROPEEN**

(45) Date de publication et mention
de la délivrance du brevet:
**19.06.2019 Bulletin 2019/25**

(51) Int Cl.:
***G16H 50/20*** *(2018.01)*

(21) Numéro de dépôt: **08702251.3**

(22) Date de dépôt: **16.01.2008**

(86) Numéro de dépôt international:
**PCT/IB2008/000089**

(87) Numéro de publication internationale:
**WO 2008/087528 (24.07.2008 Gazette 2008/30)**

(54) **PROCEDE DE SUIVI DE PATIENTS HEMODIALYSIS HYPERTENDUS.**

VERFAHREN ZUR BEHANDLUNG VON HÄMODIALYSE-PATIENTEN MIT BLUTHOCHDRUCK

METHOD OF MONITORING HYPERTENSIVE HAEMODIALYSIS PATIENTS.

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **17.01.2007 FR 0700298**

(43) Date de publication de la demande:
**25.11.2009 Bulletin 2009/48**

(73) Titulaire: **Gambro Lundia AB**
**220 10 Lund (SE)**

(72) Inventeur: **BENE, Bernard**
**F-69540 Irigny (FR)**

(74) Mandataire: **Ponzellini, Gianmarco et al**
**PGA S.P.A., Milano**
**Succursale di Lugano**
**Viale Castagnola, 21c**
**6900 Lugano (CH)**

(56) Documents cités:
**WO-A-2006/031186      US-A- 6 110 384**
**US-A1- 2004 225 201**

EP 2 122 523 B1

**Description**

Domaine technique de l'invention

La présente invention concerne la détermination de l'appartenance d'un patient soumis à des séances successives de traitement extracorporel de sang à un groupe d'hypertendus, et plus particulièrement concerne une méthode et des dispositifs innovants et améliorés pour la détermination l'appartenance d'un patient soumis à des séances successives de traitement extracorporel de sang à un groupe d'hypertendus et pour la proposer ou recommander la modification de certains paramètres pour remédier à certains effets non désirables de l'hypertension détectée.

Etat de la technique antérieure :

Le traitement extracorporel de sang est utilisé avec des patients incapables d'éliminer efficacement des matières de leur sang, par exemple dans le cas d'un patient qui souffre d'une défaillance temporaire ou permanente des reins. Ces patients et d'autres patients peuvent suivre un traitement extracorporel du sang pour ajouter ou éliminer des matières dans leur sang, pour maintenir un équilibre acide-base ou pour éliminer des fluides corporels excessifs, par exemple.

Le traitement extracorporel du sang est typiquement effectué en prélevant le sang du patient en un flux continu, en introduisant le sang dans une chambre primaire d'une unité de traitement (ou filtre) dans laquelle le sang passe à travers une membrane semi-perméable.

On appelle circuit extracorporel sanguin le circuit comprenant une aiguille pour prélever le sang par l'accès vasculaire du patient, une ligne de prélèvement ou ligne artérielle, le premier compartiment de l'unité de traitement, une ligne de retour ou ligne veineuse et une aiguille pour injecter le sang en retour par l'accès vasculaire.

La membrane semi-perméable laisse passer, de manière sélective, des matières indésirables contenues dans le sang à travers la membrane, de la chambre primaire vers la chambre secondaire, et laisse également passer, de manière sélective, des matières bénéfiques contenues dans le liquide passant dans la chambre secondaire à travers la membrane vers le sang passant dans la chambre primaire, en fonction du type de traitement.

Il y a plusieurs types de traitements extracorporels de sang. De tels traitements comprennent, par exemple, l'hémodialyse, l'hémofiltration, l'hémodiafiltration, la plasmaphérèse, l'oxygénation de sang...

Un patient peut souffrir d'une défaillance permanente des reins. Dans ce cas, il devra subir des séances régulières, par exemple trois fois par semaine, de traitement extracorporel sanguin avec un débit d'extraction de sang relativement élevé, à savoir à peu près entre 150 et 500mL/min. Ces séances sont d'une durée approximative de trois à quatre heures.

Il est connu que des patients suivants un traitement de dialyse chronique peuvent être sujets à de l'hypertension. L'hypertension est une cause majeure de décès d'origine cardiaque de patients suivants un traitement de dialyse chronique.

Il est connu une première publication scientifique sous le nom de « principles and practice of dialysis, second édition » où son auteur Willima L. Einrich recommande quelques principes de base sur le style de vie des patients dialyses hypertendus. Parmi ces principes existent ceux de modérer son alimentation en sodium, augment l'activité physique, limiter la prise quotidienne de boissons alcoolisées, éviter de fumer, éliminer l'usage de cocaine ou d'amphétamines.

Il est connu par ailleurs une deuxième publication scientifique sous le nom de « néphrologie et thérapeutique - hypertension et dialyse » d'octobre 2007, volume 3 supplément 3où ses auteurs C.Chazot et B.Charra sur les traitement non médicamenteux de l'hypertension artérielle en hémodialyse. Cette publication énonce que « la correction de l'hypertension artérielle en hémodialyse fait par méthode du poids sec qui consiste en une réduction progressive du poids de fin de dialyse jusqu'à la normalisation de la pression artérielle. Les mesures associées nécessaires sont le régime désodé et le respect d'e la balance sodée au cours de la séance, l'arrêt des hypertenseurs , la durée suffisante de la séance de dialyse et l'effort pédagogique envers le patient ». Toutefois ces paramètres sont cités comme une liste énonciative, sans différencier l'un par rapport à l'autre, et sans leur donner de priorité.

Enfin, il est connu une troisième publication dans le journal « nephrology nursing de novembre-décembre 2004, vol.31, numéro 6, par Wendy Purcell, Elisabeth Manias, Allison Williams et Rowan Walker. Selon l'articel, l'élimination d'eau en excès dans le patient pendant la séance d'hémodialyse peut normaliser la pression artérielle avec succès, sous réserve de d'arriver précisément au poids sec souhaité à la fin de la séance.

US-6110384 concerne un procédé de détermination d'un paramètre (dialysance de l'échangeur pour une substance ou concentration de la substance dans le sang) significatif du progrès d'un traitement extracorporel de sang.

Néanmoins il n'existe pas, à la connaissance du demandeur, de système permettant aujourd'hui de déterminer efficacement et automatiquement l'appartenance à un groupe de patients hypertendus soumis à des séances de traitement extracorporel de sang ni permettant de recommander la modification d'un paramètre particulier pour tenter de remédier à ce groupe détecté.

Il est donc nécessaire de suppléer à ce manque par un procédé et un appareil pour déterminer efficacement l'état d'hypertension du patient, ceci permettant une surveillance accrue de l'hypertension non souhaitable et une prise en charge plus rapide et plus en aval des inconvénients liés à une telle hypertension.

Exposé de l'invention:

L'invention concerne un système de calcul et de commande pour déterminer l'appartenance d'un patient destiné à suivre plusieurs séances (i,j) de traitement extracorporel de sang par extraction et retour du sang via l'accès vasculaire, à un groupe parmi plusieurs groupes de patients hypertendus, le système comprenant les moyens suivants:

a) des moyens pour déterminer la valeur d'au moins un paramètre représentatif ($\Delta Pi$, $\Delta Pj$...) de l'évolution de la masse ($\Delta P$) du patient inter dialytique, pour au moins deux séances (i,j...),
b) des moyens pour déterminer la valeur (CPi, CPj, ...) d'au moins un paramètre représentatif de la conductivité plasmatique (CP) du patient pour au moins deux séances (i,j),
c) des moyens pour déterminer la valeur (TMi, TMj) d'un paramètre représentatif du transfert de masse ionique du traitement pour au moins deux séances (i,j),
d) des moyens programmés pour déterminer l'appartenance à un groupe d'hypertendus du patient en fonction de l'évolution sur plusieurs séances d'au moins un des trois ensembles suivants de valeurs:

- un premier ensemble d'au moins deux valeurs ($\Delta Pi$, $\Delta Pj$) déterminées de l'évolution de la masse ($\Delta P$) du patient inter dialytique,
- un deuxième ensemble d'au moins deux valeurs (CPi, CPj...) déterminées représentatives de la conductivité plasmatique et ;
- un troisième ensemble d'au moins deux valeurs (TMi, TMj) déterminées représentatives du transfert de masse ionique.

L'invention concerne également une machine de traitement extracorporel de sang comprenant au moins:

- une unité de traitement de sang capable de mettre en oeuvre un traitement extracorporel sanguin par circulation de sang via un circuit extracorporel sanguin comprenant une ligne artérielle, une première chambre d'un filtre séparé par une membrane semi-perméable, une ligne veineuse et par circulation de dialysat dans une deuxième chambre du filtre,
- des moyens de mémorisation stockant au moins des valeurs d'au moins un paramètre représentatif (Pi, Pj...) de la masse (P) du patient, au moins des valeurs d'au moins un paramètre représentatif (CPi, CPj...) de la conductivité plasmatique du patient et des valeurs d'au moins un paramètre représentatif du transfert de masse ionique (TMi, TMj...) relatives à au moins un patient soumis à plusieurs séances (i,...j) de traitement extracorporel sanguin,
- un système de calcul et de commande selon l'invention pour la détermination de l'appartenance à un groupe d'hypertendus du patient dont les valeurs de paramètres d'au moins un paramètre représentatif (Pi, Pj...) de la masse (P) du patient, des valeurs d'au moins un paramètre représentatif (CPi, CPj...) de la conductivité plasmatique du patient et des valeurs d'au moins un paramètre représentatif du transfert de masse ionique (TMi, TMj...) sont stockées dans lesdits moyens de mémorisation.

Brève description des dessins :

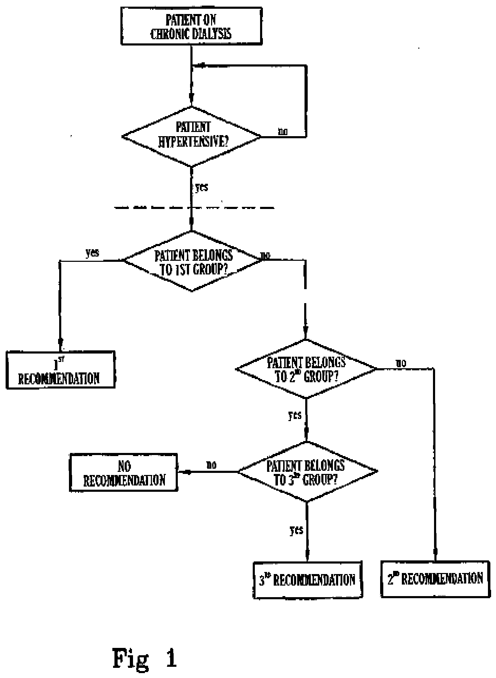

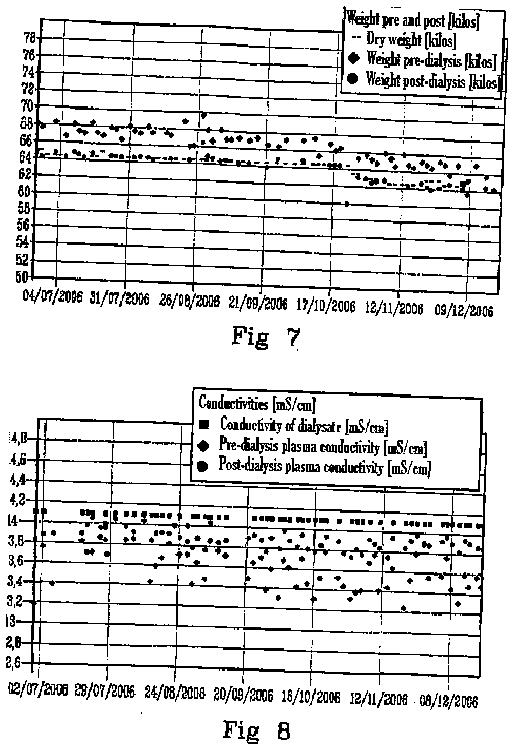

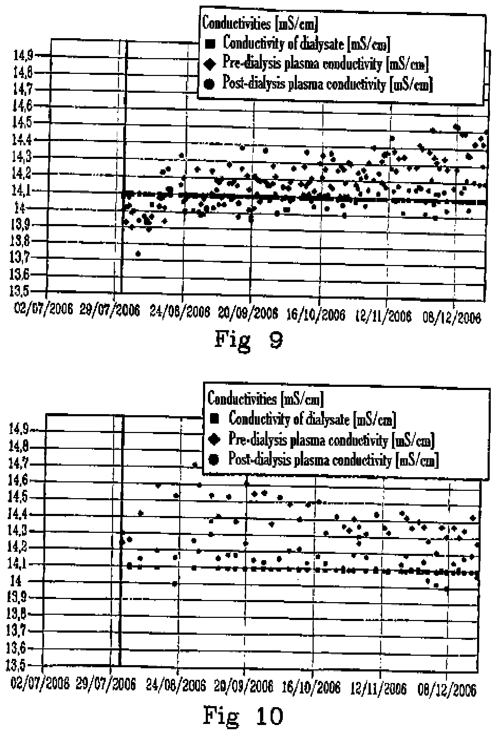

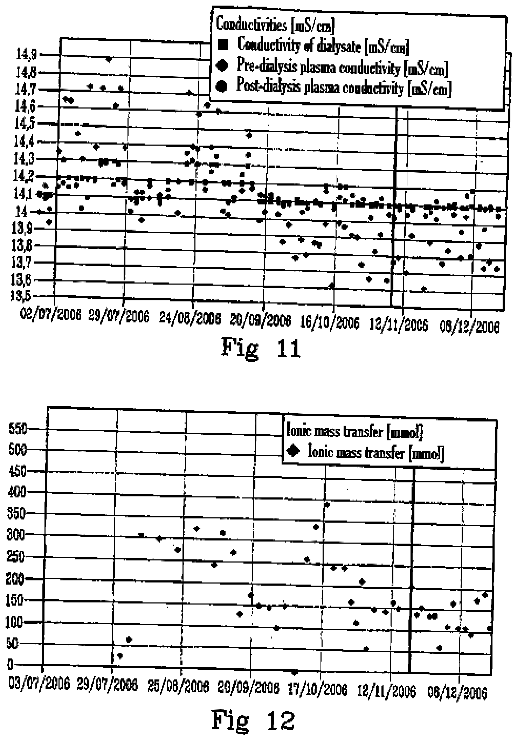

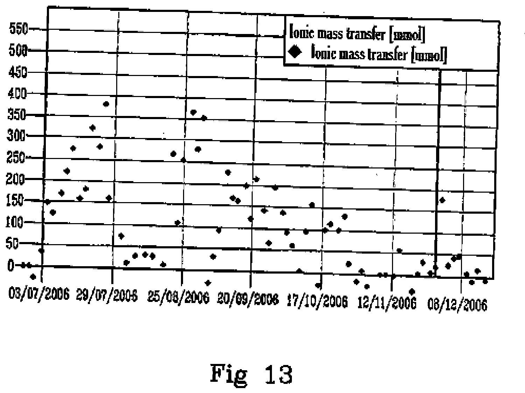

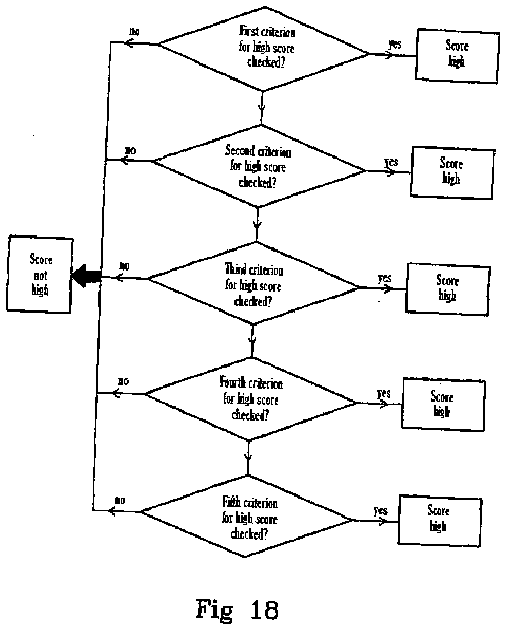

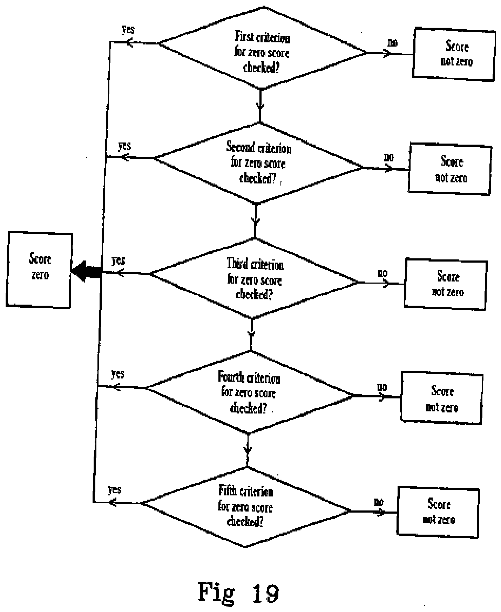

On se reportera aux dessins annexés en figure 1 à 19 sur lesquelles :

- les figures 1 et 2 représentent les étapes générales et respectivement particulières du procédé de détermination de l'appartenance d'un patient à un groupe d'hypertendus ;
- les figures 3, 4 et 5 représentent des exemples de variation de la tension d'un patient avec différentes variabilités d'un patient;
- les figures 6 et 7 représentent des exemples de variation de la prise de poids inter dialytique et de variation du poids sec d'un patient ;
- les figures 8, 9, 10 et 11 représentent des exemples de variation sur plusieurs mois de la conductivité plasmatique pré dialytique d'un patient;
- les figures 12 et 13 représentent des exemples d'évolution sur plusieurs mois du transfert de masse ionique d'un patient;
- les figures 14 et 15 représentent un schéma d'une l'installation complète logicielle et matérielle selon l'invention.
- la figure 16 représente les étapes principales du procédé de détermination du score de risque,
- la figure 17 représente une alternative de l'ordre des étapes principales du procédé de détermination du score de risque,
- la figure 18 représente les étapes de la détermination du score de risque élevé ou non,
- la figure 19 représente les étapes de la détermination du score de risque nul ou non,

Exposé détaillé de l'invention :

Un système de calcul et de commande pour déterminer l'appartenance d'un patient destiné à suivre plusieurs séances (i,j) de traitement extracorporel de sang par extraction et retour du sang via l'accès vasculaire, à un groupe parmi plusieurs groupes de patients hypertendus, le système comprenant les moyens suivants:

a) des moyens pour déterminer la valeur d'au moins un paramètre représentatif ($\Delta Pi$, $\Delta Pj$...) de l'évolution de la masse ($\Delta P$) du patient inter dialytique, pour au moins deux séances (i,j...),

b) des moyens pour déterminer la valeur (CPi, CPj, ...) d'au moins un paramètre représentatif de la conductivité plasmatique (CP) du patient pour au moins deux séances (i,j),

c) des moyens pour déterminer la valeur (TMi, TMj) d'un paramètre représentatif du transfert de masse ionique du traitement pour au moins deux séances (i,j),

d) des moyens programmés pour déterminer l'appartenance à un groupe d'hypertendus du patient en fonction de l'évolution sur plusieurs séances d'au moins un des trois ensembles suivants de valeurs:

- un premier ensemble d'au moins deux valeurs (ΔPi, ΔPj) déterminées de l'évolution de la masse (ΔP) du patient inter dialytique,
- un deuxième ensemble d'au moins deux valeurs (CPi, CPj...) déterminées représentatives de la conductivité plasmatique et;
- un troisième ensemble d'au moins deux valeurs (TMi, TMj) déterminées représentatives du transfert de masse ionique.

Pour toute l'invention, le système de calcul et de commande peut prendre la forme d'une unité de calcul et de commande analogique dument programmée, numérique dument programmée, comportant au moins un microprocesseur et au moins une mémoire dument programmée. Les mémoires pourront être une parmi ou une combinaison des mémoires suivantes : mémoire magnétique, mémoire RAM, mémoire ROM, ou toute autre mémoire envisageable par l'homme du métier.

Les moyens pour déterminer la valeur d'au moins un paramètre représentatif de l'évolution de la masse du patient inter dialytique, les moyens pour déterminer la valeur de paramètres représentatifs de la conductivité plasmatique et les moyens pour déterminer la valeur des paramètres représentatifs du transfert de masse ionique l'efficacité d'épuration peuvent être composés de:

a) au moins une mémoire contenant la valeur de ces paramètres et que les moyens programmés pourront consulter pour connaître les valeurs choisies, et/ou
b) des appareils de détection des paramètres correspondants :

∘ des détecteurs ou capteurs de poids ou de masse tels que des pesons, une balance...,
∘ des moyens pour mesurer la conductivité ou la concentration d'une substance dans le sang, par exemple des moyens pour détecter la conductivité plasmatique en sodium, qui sont connus par exemple sous le nom de DIASCAN commercialisé par GAMBRO et dont on se reportera à la description détaillée au brevet EP 0 547 025, réalisé pas des capteurs, des biocapteurs, des microcapteurs..

∘ des moyens pour mesurer le transfert de masse ionique qui peut par exemple être calculé comme suit :
TMI = Q2 * C2 -Q1 * C1, où Q1 et Q2 sont les débits ioniques du dialysat en entrée du dialyseur et respectivement en sortie du dialyseur, et C1 et C2 les concentrations ioniques en entrée du dialyseur et respectivement en sortie du dialyseur, on pourra considérer le débit et la concentration de l'ion sodium qui est représentatif de l'ensemble.

c) une unité de calcul et de commande qui sera dument programmée pour mettre en oeuvre le procédé selon l'invention.

Aussi, le Système selon l'invention peut déterminer les groupes d'hypertension qui sont divisés en un premier groupe de patients hypertendus, un deuxième groupe de patients hypertendus, et un troisième groupe de patients hypertendus.

En effet, il s'agit de diviser la population de patients hypertendus en plusieurs groupes selon l'évolution historique sur plusieurs séances de certains de leurs paramètres. L'invention peut comporter deux ou trois groupes.

Le système selon l'invention a des moyens programmés pour déterminer l'appartenance à un groupe d'hypertendus comportent des moyens programmés pour déterminer si le patient appartient au premier groupe.

Le système selon l'invention a des moyens programmés pour déterminer l'appartenance à un groupe d'hypertendus comportent des moyens programmés pour déterminer si le patient appartient au deuxième groupe lorsque le patient n'appartient pas au premier groupe.

Le système selon l'invention a des moyens programmés pour déterminer l'appartenance à un groupe d'hypertendus comportent des moyens programmés pour déterminer si le patient appartient au troisième groupe lorsque le patient n'appartient pas au deuxième groupe.

Dans le système selon l'invention, la valeur représentative (ΔPi, ΔPj...) de l'évolution inter dialytique de la masse (ΔP) du patient, pour au moins deux séances (i,j...) est choisie parmi le groupe comprenant:

- la prise de poids du patient entre la fin d'une séance (i) et le début de la séance suivante (i+1),
- la prise de masse du patient entre la fin d'une séance (i) et le début de la séance suivante (i+1),
- un paramètre proportionnel à un des précités paramètres.

Dans le système selon l'invention, le paramètre représentatif de la conductivité plasmatique est égale ou fonction d'au moins un des paramètres suivants :

- la conductivité plasmatique du patient,
- la conductivité plasmatique prédialytique du patient c'est-à-dire la conductivité plasmatique avant la séance de traitement extracorporel de sang,
- un paramètre proportionnel à un des précités paramètres.

Dans le système selon l'invention, le ou les paramètres extracorporels ont été mesurés pour une séance de traitement extracorporel sanguin qui consiste à circuler le sang du patient à un débit extracorporel sanguin et dans un circuit extracorporel sanguin, ce circuit comprenant une ligne artérielle où existe une pression artérielle, un filtre et une ligne veineuse où existe une pression veineuse. Par ailleurs, le circuit filtrat ou dialysat consiste à extraire du filtre un filtrat par une ligne de vidange ou mettre du dialysat en contact avec le sang dans le filtre séparé par une membrane semiperméable dans le circuit dialysat comportant une ligne d'amenée du dialysat vers le filtre et une ligne de vidange du dialysat usé hors du filtre. La conductivité plasmatique est la conductivité du plasma du patient.

Dans le système selon l'invention, le paramètre représentatif du transfert de masse ionique pour au moins deux séances (i,j), est égal ou fonction d'au moins un des paramètres suivants :

- le transfert de masse ionique pendant la séance,
- le transfert de masse de sodium pendant la séance du patient,
- un paramètre proportionnel à un des précités paramètres.

Les valeurs du ou des paramètres représentatifs du transfert de masse ionique par exemple peuvent être des valeurs moyennes de ce paramètre sur une séance de traitement. Ces valeurs peuvent alternativement être une valeur instantanée choisie à un moment t de la séance, au début, au milieu ou à la fin de la séance, ou peuvent être encore une valeur médiane, ou toute autre valeur mathématique représentative le plus exactement possible du paramètre ou de son évolution sur une séance de dialyse.

Les valeurs du ou des paramètres représentatifs de la conductivité plasmatique par exemple peuvent être des valeurs de la conductivité plasmatique avant la séance, plus particulièrement au début de la séance de traitement.

On comprendra que la mise en oeuvre du procédé selon l'invention est bien séparée du traitement extracorporel sanguin.

Le système selon l'invention peut comporter des moyens pour déterminer la tension du patient. Cette tension peut être la tension artérielle (préférentiellement), la tension systolique, la moyenne de la tension artérielle et tension systolique. Cette tension peut être prise sur le patient couché, avant le début de la séance.

Dans le système selon l'invention, le patient est initialement considéré hypertendu lorsque les valeurs représentatives de sa tension augmentent d'au moins environ 20mmHg sur au moins deux séances et/ou la valeur représentative de sa tension pour une séance est supérieure à environ 150mmHg ou supérieure à 150mmHg. Préférentiellement la condition initiale de patient hypertendu pourra être d'autant plus vérifiée quand additionnellement l'augmentation des valeurs représentatives de sa tension reste stable sur la durée considérée. Pour vérifier si l'évolution de la tension reste stable, on peut utiliser des méthodes mathématiques connues, par exemple la méthode des moindres carrés. Ceci est valable pour l'évolution de toutes les valeurs représentatives selon l'invention.

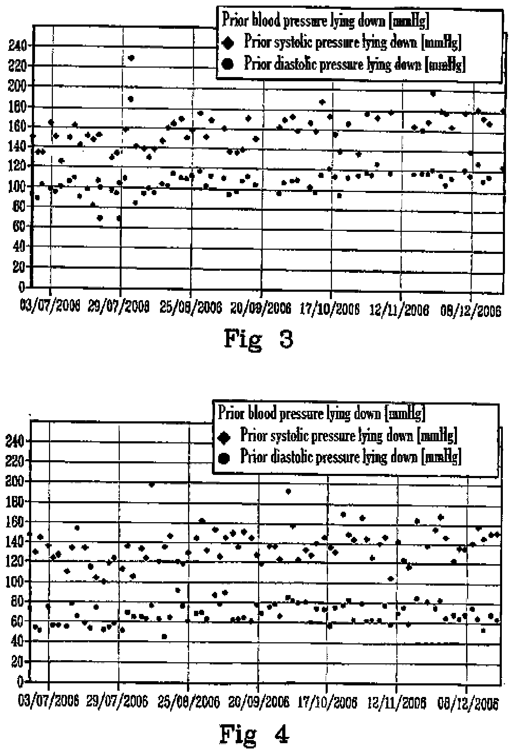

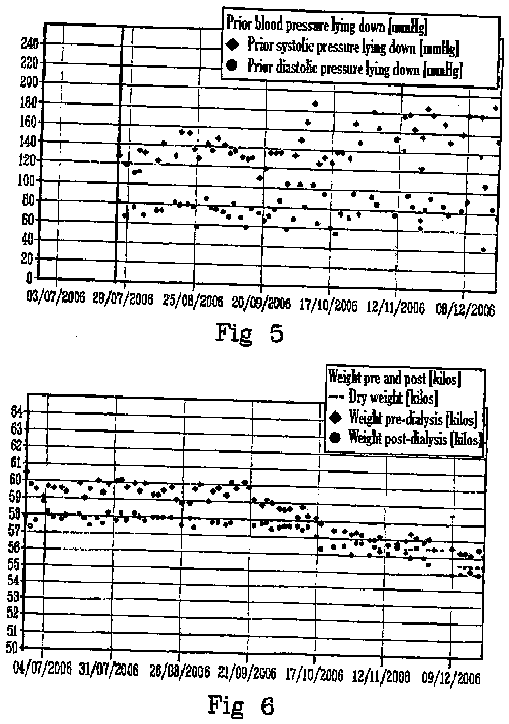

En effet, comme illustrés en figures 3, 4 et 5, on constate pour trois patients différents que la stabilité e l'évolution est différente. Sur la figure 3, pour la courbe de pression artérielle du patient couché avant la séance (courbe inférieure), la variabilité est faible, et l'évolution regardée sera d'autant plus prise en considération. Sur les figures 4 et 5, pour la courbe de pression artérielle du patient couché avant la séance (courbe inférieure), la variabilité est importante, et l'évolution regardée sera d'autant moins prise en considération.

Dans le système selon l'invention, les moyens programmés pour déterminer si le patient appartient au premier groupe d'hypertendus sont des moyens programmés pour déterminer si les valeurs représentatives de l'évolution de masse du patient inter dialytique ($\Delta P$) sur plusieurs séances (i,j) montrent une tendance de diminution, le patient étant considéré comme appartenant au premier groupe quand la tendance est à la diminution.

On a une tendance à la diminution du paramètre mesuré, lorsque la courbe générale de l'ensemble des valeurs montre une diminution. Cette courbe peut comporter quelques valeurs qui ne rentrent pas dans la tendance (environ 20% des valeurs) sans que celles-ci ne soient tenues en compte. Le patient peut en effet être en mauvaise forme lors d'une séance ou le paramètre peut avoir une mesure erronée.

Dans le système selon l'invention, les moyens de détermination d'appartenance au premier groupe d'hypertendus considéreront l'appartenance au premier groupe lorsque l'évolution des valeurs représentatives de la masse est supérieure à environ un kilogramme.

Dans le système selon l'invention, les moyens programmés pour déterminer si le patient appartient au deuxième groupe d'hypertendus sont des moyens programmés pour déterminer si les valeurs représentatives de la conductivité plasmatique (CP) sur plusieurs séances (i,j) montrent une tendance à l'augmentation ou additionnellement une tendance à la diminution, le patient étant considéré comme appartenant au deuxième groupe quand la tendance est à l'augmentation, ou additionnellement à une faible diminution.

Dans le système selon l'invention, les moyens pour déterminer si le patient appartient au deuxième groupe d'hypertendus considéreront l'appartenance au deuxième groupe lorsque les valeurs représentatives de la conductivité plasmatique augmentent ou lorsque les valeurs représentatives de la conductivité plasmatique diminuent de moins d'environ 0,3mS/cm.

Le critère d'appartenance au deuxième groupe est additionnellement une évolution des valeurs représentatives de la conductivité plasmatique qui reste stable.

Pour la détermination de l'appartenance au deuxième groupe, l'invention propose alternativement un :

un système où les moyens programmés pour déterminer la non appartenance au deuxième groupe d'hypertendus sont des moyens programmés pour déterminer si les valeurs représentatives de la conductivité plasmatique (CP) sur plusieurs séances (i,j) montrent une tendance à la diminution.

Ce système comporte des moyens programmés pour déterminer la non appartenance au deuxième groupe d'hypertendus considéreront la non appartenance au deuxième groupe lorsque les valeurs représentatives de la conductivité plasmatique diminuent d'au moins environ 0,3mS/cm.

En effet, dans le cas où les valeurs représentatives de la conductivité plasmatique (CP) sur plusieurs séances (i,j) répondent à cette tendance à la diminution, le patient sera considéré comme n'appartenant au deuxième groupe. Dans le cas contraire, le patient sera considéré comme appartenant au deuxième groupe.

Le critère de non appartenance au deuxième groupe est additionnellement rempli quand une diminution des valeurs représentatives de la conductivité plasmatique qui est stable.

Dans le système selon l'invention, ù les moyens programmés pour déterminer l'appartenance au troisième groupe d'hypertendus sont des moyens programmés pour déterminer si les valeurs représentatives du transfert de masse ionique (TM) sur plusieurs séances (i,j) montre une tendance de diminution, le troisième niveau étant considéré rempli quand la tendance est à la diminution.

Système selon la revendication précédente où les moyens de détermination de l'appartenance au troisième groupe d'hypertendus considéreront l'appartenance au troisième groupe lorsque les valeurs représentatives de la conductivité plasmatique diminuent d'au moins environ 200mmol/séance.

Un critère supplémentaire serait que l'évolution de la conductivité plasmatique reste stable ou montre une tendance stable.

Pour toute l'invention, les moyens de détermination peuvent être des codes logiciels qui peuvent se présenter sous forme de codes sources ou de codes exécutables directement sur un processeur. Les moyens pour déterminer l'appartenance à un groupe de patients hypertendus peuvent être des sous-routines des moyens de détermination de score.

Dans le système selon l'invention, pour la détermination de l'appartenance à un des trois groupes d'hypertendus, si le nombre de séances considérées est supérieur à deux, alors les moyens de détermination correspondant sont destinés à opérer avec au moins deux séances successives (i,i+1).

Dans le système selon l'invention, pour la détermination de l'appartenance à un des trois groupes d'hypertendus, si le nombre de séances considérées est supérieur à deux, alors les moyens de détermination correspondant sont destinés à opérer en identifiant la première séance temporelle considérée comme séance antérieure et en identifiant la dernière séance temporelle considérée comme séance postérieure.

le système selon l'invention, pour la détermination de l'appartenance à un des trois groupes d'hypertendus, si le nombre de séances considérées est supérieur à deux, alors les moyens de détermination correspondant sont destinés à considérer comme séances temporelles celles pour lesquelles les valeurs d'au moins un paramètre considéré sont les plus éloignées.

Espacement des séances considérées :

Pour l'appartenance aux groupes d'hypertendus, entre une séance antérieure (i) et une séance postérieure (j), il peut se passer, en terme de nombre de séances, une dizaine voire plusieurs dizaines de séances, ou, en terme de semaines (à environ 3 séances par semaine), il peut se passer entre 2 semaines et 8 semaines, voire même entre 2 et 6 mois.

Généralement, les appartenances aux groupes sont déterminées en fonction du même ensemble de séances. Cet ensemble de séances pourra s'étaler entre 2 semaines et 6 mois, 3 semaines étant habituellement choisi, ou encore ce nombre de séances pourra être compris entre environ une dizaine et plusieurs dizaines.

Généralement, néanmoins, il ne sera pas nécessaire de mettre en oeuvre le procédé de détermination selon l'invention à chaque fin de séance de traitement. L'utilisateur pourra trouver un compromis efficace de suivi en mettant en oeuvre le procédé de l'invention chaque trois séances (à peu près chaque semaine donc) par exemple, ou bien chaque six séances (à peu près chaque quinzaine donc).

Le système selon l'invention peut comprendre des moyens programmés pour recommander ou contrôler la modification d'au moins un paramètre médical ou d'au moins un paramètre machine pour chaque groupe d'hypertendus. La recommandation pourra être faite grâce à des moyens d'interface couplés au système de calcul et de commande, qui peuvent être par exemple un écran. Le contrôle pourra être fait par le système de calcul et de commande relié au matériel nécessaire.

Le système selon l'invention peut comprendre des moyens programmés pour, lorsque l'appartenance au premier groupe d'hypertendus est considérée, proposer ou contrôler lors d'une future séance de diminuer la masse du patient devant être atteinte en fin de séance.

Cette masse (ou par équivalence poids ou tout autre paramètre proportionnel à ceux-ci) est appelé « poids sec » : c'est le poids, fixé généralement en début de séance de dialyse, que le patient doit atteinte à la fin de la séance. Généralement on mesure le poids du patient en début de séance, et, en fonction du poids sec à atteindre, on commande les moyens d'ultrafiltration (par exemple pompe d'ultrafiltration en aval du filtre sur le côté dialysat, extrayant du liquide depuis le circuit extracorporel) et éventuellement les moyens d'injection de liquide (pompe sur ligne d'injection en pré-filtre et/ou pompe sur ligne d'injection en post-filtre) en fonction de la quantité de liquide injectée et quantité extraite connue par exemple par calcul ou par mesure en direct grâce à des pesons du liquide injecté et du liquide extrait du circuit extracorporel).

Le système selon l'invention peut comprendre les moyens commandés pour proposer ou contrôler ladite diminution de la masse patient sont commandés pour que la diminution de la valeur représentative de la masse soit d'au moins environ 0,5 kg, voire environ 1kg.

Le système selon l'invention peut comprendre des moyens programmés pour, lorsque l'appartenance au deuxième groupe d'hypertendus est considérée, proposer la diminution de la quantité de sel injecté par le patient d'une séance à l'autre.

Cette diminution de sel se fera par la prescription au patient d'un régime sans sel ou avec peu de sel, que le médecin détermine au mieux selon le patient.

Le système selon l'invention peut comprendre des moyens programmés pour, lorsque l'appartenance au troisième groupe d'hypertendus est considérée, proposer ou contrôler lors d'une future séance de diminuer la valeur représentative de la conductivité dialytique, à savoir la conductivité du liquide de dialyse utilisée pendant la séance de traitement.

Le système selon l'invention, la diminution proposée ou contrôlée de la conductivité dialytique est d'au moins environ 0,2mmol. Préférentiellement, la conductivité dialytique sera diminuée de 0.2mmol par séance d'une séance à l'autre sur plusieurs séances (on n'envisage pas efficacement de diminuer la conductivité d'une trop grande amplitude en une seule fois).

Dans le système selon l'invention, les séances de traitement considérées s'étalent sur plusieurs séances s'étalant sur une durée d'environ six mois, préférentiellement environ 3 mois, voire environ 1 mois.

Selon un exemple, un ordinateur comprend :

- des moyens de mémorisation stockant au moins des valeurs d'au moins un paramètre représentatif (Pi, Pj...) de la masse (P) du patient, au moins des valeurs d'au moins un paramètre représentatif (CPi, CPj...) de la conductivité plasmatique du patient et des valeurs d'au moins un paramètre représentatif du transfert de masse ionique (TMi, TMj...) relatives à au moins un patient soumis à plusieurs séances (i,...j) de traitement extracorporel sanguin,

- un système de calcul et de commande selon l'invention pour la détermination de l'appartenance à un groupe d'hypertendus, du patient dont les valeurs de paramètres d'au moins un paramètre représentatif (Pi, Pj...) de la masse (P) du patient, les valeurs d'au moins un paramètre représentatif de la conductivité plasmatique (CP) et des valeurs d'au moins un paramètre représentatif du transfert de masse ionique sont stockées dans lesdits moyens de mémorisation.

L'invention concerne aussi une machine de traitement extracorporel de sang comprenant au moins:

- une unité de traitement de sang capable de mettre en oeuvre un traitement extracorporel sanguin par circulation de sang via un circuit extracorporel sanguin comprenant une ligne artérielle, une première chambre d'un filtre séparé par une membrane semi-perméable, une ligne veineuse et par circulation de dialysat dans une deuxième chambre du filtre,

- des moyens de mémorisation stockant au moins des valeurs d'au moins un paramètre représentatif (Pi, Pj...) de la masse (P) du patient, au moins des valeurs d'au moins un paramètre représentatif (CPi, CPj...) de la conductivité plasmatique du patient et des valeurs d'au moins un paramètre représentatif du transfert de masse ionique (TMi, TMj...) relatives à au moins un patient soumis à plusieurs séances (i,...j) de traitement extracorporel sanguin,

- un système de calcul et de commande selon l'invention pour la détermination de l'appartenance à un groupe d'hypertendus du patient dont les valeurs de paramètres d'au moins un paramètre représentatif (Pi, Pj...) de la masse (P) du patient, des valeurs d'au moins un paramètre représentatif (CPi, CPj...) de la conductivité plasmatique du patient et des valeurs d'au moins un paramètre représentatif du transfert de masse ionique (TMi, TMj...) sont stockées dans lesdits moyens de mémorisation.

Selon un exemple, un réseau comprend :

- un serveur,
- au moins une machine de traitement de sang reliée au serveur, chaque machine comprenant :

  - des moyens de mesure et/ou de calcul de données médicales relatives à au moins un paramètre représentatif (Pi, Pj...) de la masse du patient, au moins un paramètre représentatif (CPi, CPj...) de la conductivité plasmatique du patient et au moins un paramètre représentatif du transfert de masse ionique (TMi, TMj...),

  - des moyens d'émission d'au moins une partie de ces données mesurées et/ou calculées vers le serveur,

- le serveur comprenant :

  - des moyens de réception d'au moins une partie des données médicales relatives à des traitements extracorporels de sang,
  - des moyens de mémorisation pour stocker les données reçues par les moyens de réception depuis une ou plusieurs machines de traitement de sang,
  - un système de calcul et de commande selon l'invention, destiné à opérer à partir desdites données reçues,

- au moins un poste_(poste client par exemple) capable de communiquer avec le serveur pour recevoir au moins les résultats de la mise en oeuvre dudit système de calcul et de commande.

Le réseau pouvant comprendre une unité d'affichage des résultats de groupe d'hypertendus déterminé.

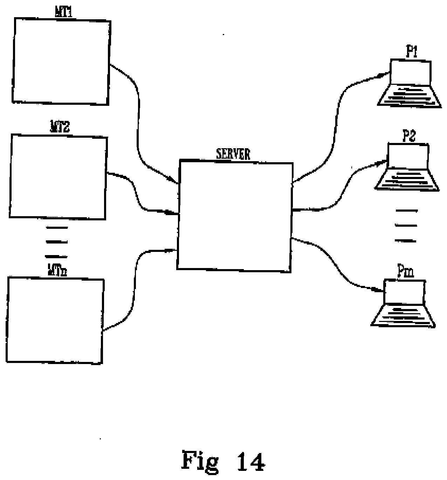

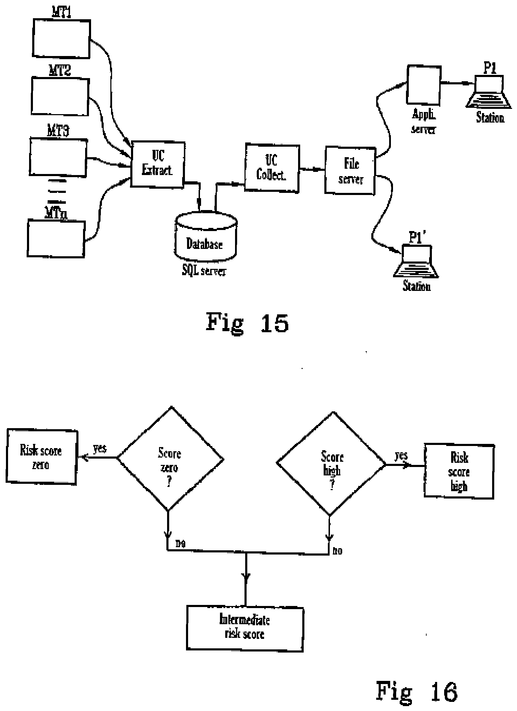

Selon les figures 14 et 15, des exemples de réseau sont représentés. Ces réseaux peuvent comporter une partie d'éléments en salle de traitement, une autre partie dans le bureau du médecin ou bien dans un autre endroit où un coordinateur travaille à distance pour une ou plusieurs cliniques.

Sur la figure 14, il est schématisé un ensemble de machines de traitement MT1, Mt2,...MTn qui sont placées dans une clinique et qui sont chacune reliée par une connexion filaire ou par une connexion sans fil à un serveur.

Ce serveur est capable de recevoir les données mesurées ou calculées pendant et/ou après les séances de traitement, est capable de mémoriser tout ou partie des données produites par la machine de traitement. Lorsqu'une partie des données seulement est mémorisée, elle peut avoir été extraite par un logiciel de collecte spécifique de ces données. Ce logiciel de collecte spécifique peut se trouver dans la machine de traitement ou dans le serveur. Ceci permet de réduire la quantité de données à stocker qui est très élevée due au nombre de patients, de paramètres et de séances enregistrées.

Une fois les paramètres nécessaires sélectionnés, le serveur met en oeuvre le procédé selon l'invention, ce procédé étant automatisé par la mise en oeuvre d'un logiciel expert.

L'utilisateur pourra accéder au moins aux résultats du procédé selon l'invention via un poste relié audit serveur. Les liaisons décrites peuvent être sécurisées par des techniques connues pour des raisons de confidentialité des données relatives à un patient. Alternativement ou additionnellement, les données peuvent être, avant émission, « anonymisées » par l'attribution d'un code à chaque patient, sans faire apparaître le nom du patient lors des échanges de données.

Un procédé de détermination de l'état de l'appartenance à un groupe d'hypertendus d'un patient destiné à suivre des séances successives (i,j) de traitement extracorporel de sang par extraction et retour du sang, le procédé de détermination comprenant les étapes suivantes:

a) déterminer la valeur d'au moins un paramètre représentatif (Pi, Pj...) de la masse (P) du patient pour au moins deux séances (i,j...),
b) déterminer la valeur (CPi, CPj, ...) d'au moins un paramètre représentatif de la conductivité plasmatique (CP) du patient pour au moins deux séances (i,j),
c) déterminer la valeur (TMi, TMj) d'au moins un paramètre représentatif du transfert de masse ionique du traitement pour au moins deux séances (i,j),
d) déterminer l'appartenance à un groupe d'hypertendus du patient en fonction de l'évolution sur plusieurs séances d'au moins un des trois ensembles suivants de valeurs:

- un premier ensemble d'au moins deux valeurs (Pi, Pj) déterminées représentatives de l'évolution de la masse (ΔP) du patient inter dialytique,
- un deuxième ensemble d'au moins deux valeurs (CPi, CPj...) déterminées représentatives de la conductivité plasmatique et;
- un troisième ensemble d'au moins deux valeurs (TMi, TMj) déterminées représentatives du transfert de masse ionique.

Ce procédé préférentiellement mis en oeuvre automatiquement peut être conduit sur place dans la salle de traitement ou à distance dans une salle de la clinique ou d'un centre de traitement de données.

Selon un exemple, un programme d'ordinateur pour la détermination de l'appartenance à un groupe d'hypertendus d'un patient, programme chargeable dans la mémoire interne d'un ordinateur, comprenant des portions de code de programme d'ordinateur pour, lorsque le programme est exécuté par l'ordinateur, mettre en oeuvre le procédé selon l'invention.

Ce programme peut être enregistré sur un support lisible dans un ordinateur, le support étant une mémoire de données optique et/ou magnétique ou un support de mémoire volatile.

Avantages de l'invention :

L'invention apporte un maximum d'avantages dont les principaux sont listés ici:

- rapidité de la détermination d'un groupe de patients hypertendus,
- mis en oeuvre de l'invention sans matériel supplémentaire (médical, matériel, informatique... nécessaire ;
- temps de traitement ou d'intervention supplémentaire sur le patient épargnés ;
- coûts supplémentaires épargnés de mains d'oeuvre, d'appareils médicaux consommables, de matériel (usage du Doppler...)

- mis en oeuvre de l'invention sans manipulation supplémentaire pendant le traitement, et sans intervention pendant les séances de dialyse, donc sans produire de perturbations,
- suivi à distance de plusieurs patients dont l'hypertension est détectée et/ou dans une ou plusieurs cliniques,
- suivi à distance par un médecin d'un malade dialyse à domicile,
- anticipation de l'état à risque d'un patient hypertendu avant le début d'une séance de dialyse,
- le médecin peut se voir envoyer directement et confidentiellement les groupes déterminés.

Par ailleurs un système de calcul et de commande capable de suivre l'accès vasculaire de tout patient, y compris les patients non hypertendus, a été décrit entièrement dans la demande FR0700298 et la demande PCT/IB2007/000958 au nom de la demanderesse et incorporées ici à titre de référence. Néanmoins il est rappelé que l'invention peut revêtir la forme combinée d'un système et/ou d'un procédé pour à la fois déterminer l'appartenance à un groupe de patient hypertendus et déterminer un score de risque concernant l'accès vasculaire du patient, en fonction de l'historique de valeurs sur plusieurs séances de traitement. Toutes les remarques faites pour la détermination d'appartenance à un groupe de patients hypertendus restent valables pour la facette de détermination de l'état d'un accès vasculaire.

Le système de calcul et de commande pour la détermination de l'état d'un accès vasculaire d'un patient destiné à suivre des séances successives (i,j) de traitement extracorporel de sang par extraction et retour du sang via l'accès vasculaire comprend les moyens suivants:

a) des moyens pour déterminer la valeur (Pli, P1j, P2i, P2j, ...) d'au moins un paramètre (P1, P2...) extracorporel hémodynamique du patient pour au moins deux séances (i,j),

b) des moyens pour déterminer la valeur (Ei, Ej) de l'efficacité d'épuration du traitement pour au moins deux séances (i,j),

c) des moyens programmés pour déterminer un score de risque concernant l'état de l'accès vasculaire du patient en fonction desdites au moins deux valeurs (Pi, Pj) du paramètre extracorporel hémodynamique et desdites au moins deux valeurs (Ei, Ej) de l'efficacité d'épuration déterminées.

Les moyens pour déterminer la valeur d'au moins un paramètre extracorporel hémodynamique et les moyens pour déterminer la valeur de l'efficacité d'épuration peuvent être composés de:

d) au moins une mémoire contenant la valeur de ces paramètres et que les moyens programmés pourront consulter pour connaître les valeurs choisies, et/ou

**e)** des appareils de détection des paramètres correspondants : des détecteurs ou capteurs de pression, des moyens pour mesurer la conductivité ou la concentration d'une substance dans le sang, des moyens pour mesurer la dialysance, la dose de dialyse, la clairance,

**f)** une unité de calcul et de commande qui sera dûment programmée pour mettre en oeuvre le procédé selon l'invention.

Le score de risque peut prendre trois valeurs :

sO) score de risque nul (0) pour un patient dont l'état de l'accès vasculaire est normal,
s1) score de risque intermédiaire (1) pour un patient dont l'état de l'accès vasculaire est douteux,
s2) score de risque élevé (2) pour un patient dont l'état de l'accès vasculaire est alarmant.

Ainsi la classification du score de risque de l'accès vasculaire peut prendre trois valeurs différentes et donne un aperçu des patients à traiter immédiatement par le médecin, des cas sans problème pour lequel les séances devraient être conduites sans modification jusqu'à la prochaine détermination, et des cas douteux ou mitigés à surveiller ou pour lesquels il faut approfondir l'étude des courbes mesurées lors des dernières séances. Le médecin, suivant plusieurs dizaines de patients en même temps, pourra donc être orienté, à la lecture des résultats du système, très rapidement vers un patient nécessitant une vérification ou une intervention sur son accès vasculaire.

Diagramme principal de décision :

Le système selon l'invention a les moyens programmés pour déterminer le score de risque comportant des moyens programmés pour déterminer si le score de risque est élevé. Le système selon l'invention a les moyens programmés pour déterminer le score de risque comportant des moyens programmés pour déterminer si le score de risque est nul.

Le système selon l'invention a les moyens programmés pour déterminer le score de risque comportant des moyens programmés pour, dans le cas où le score de risque n'est déterminé ni élevé ni nul, considérer le score de risque comme intermédiaire.

Le système comprend les moyens programmés pour déterminer le score de risque qui sont programmés pour déterminer si le score de risque est élevé avant de déterminer si le score de risque est nul.

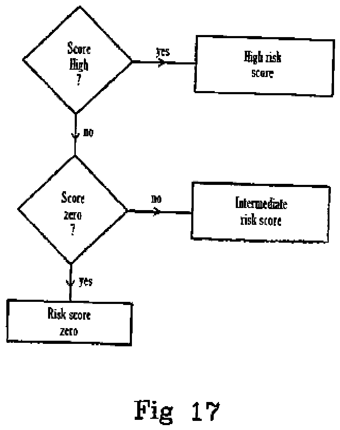

Comme représenté dans le diagramme des figures 16 et 17, l'invention peut comporter:

- la détermination du score de risque comporte l'étape de déterminer si le score de risque est élevé,

- la détermination du score de risque comporte l'étape additionnelle de déterminer si le score de risque est **nul,**
- l'étape additionnelle suivante : si le score de risque n'est déterminé ni élevé ni nul, alors il est considéré intermédiaire.

Selon une première alternative proposée dans le diagramme de la figure 16, les étapes de détermination du score élevé ou non et détermination du score nul ou non peut être mises en oeuvre sans condition temporelle, simultanément ou non. Selon une deuxième alternative proposée dans le diagramme de la figure 17, l'étape de déterminer si le score de risque est nul est effectuée après l'étape de déterminer si le score de risque est élevé.

Paramètres en jeu :

Dans le système selon l'invention, le ou les paramètres extracorporels hémodynamiques (P) ont été mesurés pour une séance de traitement extracorporel sanguin qui consiste à circuler le sang du patient à un débit extracorporel sanguin et dans un circuit extracorporel sanguin, ce circuit comprenant une ligne artérielle où existe une pression artérielle, un filtre et une ligne veineuse où existe une pression veineuse. Ces paramètres extracorporels hémodynamiques sont choisis parmi le groupe comprenant:

- Pression veineuse(Pv) extracorporelle,
- Pression artérielle (Pa) extracorporelle,
- débit sang extracorporel du patient(Qb),
- un paramètre proportionnel à un des trois précités paramètres.

Les paramètres extracorporels hémodynamiques selon l'invention sont définis comme se rapportant à la mécanique de la circulation sanguine du système cardiovasculaire extracorporel.
Les pressions seront mesurées par des capteurs de pression positionnés sur la ligne artérielle et la ligne veineuse, le débit sang pourra être considéré comme le débit imposé d'une pompe (péristaltique par exemple) positionnée sur la ligne artérielle, et/ou pourra être mesuré par un débitmètre sur cette ligne.
Ces paramètres extracorporels hémodynamiques peuvent changés du fait de l'accès vasculaire initial du patient : par exemple, plus le calibre de la fistule (ou veine centrale...) est réduit, plus le régime de pression réagit comme devant un accès vasculaire difficile.
Ces paramètres extracorporels hémodynamiques peuvent changés aussi d'une séance à l'autre si les mêmes moyens de traitement ne sont pas utilisés d'une séance à l'autre. Il est en effet recommandé de standardiser les conditions de ponction et de traitement en utilisant un hémodialyseur identique, le même accès sur le bras, la même fistule, la même aiguille ou le même diamètre d'aiguille...afin de renforcer la fiabilité du procédé. En effet, la recirculation dans la fistule peut dépendre, entre autres, du débit sanguin extracorporel, de la position de l'aiguille insérée dans la fistule, du degré de sténose de la fistule, il faut donc effectuer des séances avec des pratiques aussi régulières que possible.
Dans le système, l'efficacité d'épuration (E) peut avoir été mesurée pour au moins une séance de traitement extracorporel sanguin qui consiste à circuler le sang du patient à un débit extracorporel sanguin et dans un circuit extracorporel sanguin, ce circuit comprenant une ligne artérielle extracorporelle, un filtre et une ligne veineuse extracorporelle, l'efficacité d'épuration (E) étant égale ou fonction d'au moins un des paramètres suivants :

- la dialysance (D), ou
- la clairance (C), ou
- la concentration d'une substance contenue dans le sang avant le filtre (Cbin), ou
- la concentration d'une substance contenue dans le sang après le filtre (Cbout) dans le circuit extracorporel, ou
- la dose de dialyse (KT/v) dans des conditions de durées de séance égales, ou
- un paramètre proportionnel à un des cinq précités paramètres.

Pour l'homme du métier, tout paramètre physique ou chimique donnant une indication sur l'efficacité du transfert à travers la membrane sera pris en compte.
Il faut préciser que l'efficacité d'épuration n'est pas nécessairement calculée pendant le traitement extracorporel sanguin ni pendant la mise en oeuvre du procédé selon l'invention.
Un mode de réalisation peut avoir cette efficacité qui, une fois calculée, sera mémorisée dans des moyens adéquats. Il y aura ensuite, après la séance de traitement par exemple, pendant la mise en oeuvre du procédé selon l'invention, accès aux valeurs mémorisées.
Un mode alternatif peut être le calcul de l'efficacité en traitement, cette efficacité calculée étant utilisée aussitôt pour le procédé selon l'invention.
On comprendra que la mise en oeuvre du procédé selon l'invention est bien séparée du traitement extracorporel sanguin.
Tout ceci est valable également pour les paramètres hémodynamiques extracorporels.

Dialysance et la clairance:

On définit la dialysance (D) d'un soluté comme la masse de soluté extraite du sang par unité de temps divisée par la différence entre la concentration de ce soluté dans le sang et de ce soluté dans le liquide de dialyse, à l'entrée du dialyseur ou filtre. Cette définition s'applique en général lorsque le soluté est présent dans le sang et dans le liquide de dialyse frais (avant passage dans le filtre et contact avec le sang via la membrane semi-perméable), ou quand le soluté est présent dans le sang seulement. On parlera par exemple dans le premier cas de dialysance de sodium, de calcium ou par exemple dans le deuxième cas de dialysance d'urée ou de beta-2 microglobuline. La clairance d'un soluté est un cas particulier de la dialysance d'un soluté. C'est la dialysance lorsque le soluté est présent dans le sang seulement et par conséquent est absent du liquide de dialyse frais : on parlera de clairance d'urée.

La dialysance ou clairance d'un soluté peut être calculée de différentes façons : en ligne dans le circuit extracorporel pendant le traitement ou après le traitement, in vivo pendant le traitement ou après le traitement, en une fois ou en plusieurs fois par échantillons périodiques...

Le brevet EP 0547025, incorporé ici à titre de référence, explique un mode de calcul de la dialysance parmi d'autres. Pour mémoire, il s'agit d'une méthode pour déterminer une concentration d'une substance dans le sang d'un patient subissant un traitement de dialyse dans un rein artificiel (ou filtre ou dialyseur) et/ ou la dialysance effective pour ladite substance du rein artificiel, le rein artificiel comprenant un circuit de sang extracorporel connecté à un dialyseur ayant une membrane semi-perméable qui délimite un premier compartiment pour la circulation du sang et un deuxième compartiment pour la circulation d'un liquide de dialyse sur l'autre face de la membrane, caractérisé par les étapes de :

- mise en circulation successivement dans le deuxième compartiment du dialyseur, d'un premier et d'un deuxième liquide différant seulement par la concentration de la substance,
- mesure, dans les premier et deuxième liquides de dialyse, de la conductivité ou de la concentration de la substance en amont et en aval du dialyseur, et
- calcul, à partir de la conductivité (par un conductimètre) ou de la concentration mesurée de la substance dans les premier et deuxième liquides de dialyse, de la concentration de la substance dans le sang à l'entrée du dialyseur et/ou de la dialysance effective du rein artificiel.

Dose de dialyse:

La dose totale de dialyse délivrée est l'intégrale des valeurs de clairance ou dialysance moyenne mesurée sur un intervalle de temps déterminé.

La dose de dialyse administrée après un temps t de traitement, peut être assimilée, d'après les travaux de Sargent et Gotch, au rapport sans dimension Kt/V, où K est la clairance réelle pour l'urée, t le temps de traitement écoulé, et V le volume de distribution de l'urée, c'est-à-dire le volume d'eau total du patient (Gotch FA, Sargent SA. A mechanistic analysis of the National Coopérative Dialysis Study (NCDS). Kidney int 1985 ; 28 : 526-34).

Le brevet EP0920877, incorporé ici à titre de référence, explique un autre mode de calcul d'un paramètre représentatif de l'efficacité du traitement, par exemple la dialysance, la clairance, la dose de dialyse ou autre paramètre représentatif de l'efficacité d'un traitement extracorporel de sang.

Dans le système selon l'invention, les valeurs du ou des paramètres extracorporels hémodynamiques et de l'efficacité d'épuration peuvent être des valeurs moyennes de ces paramètres sur une séance de traitement.

Ces valeurs peuvent alternativement être une valeur instantanée choisie à un moment t de la séance, au début, au milieu ou à la fin de la séance, ou peuvent être encore une valeur médiane, ou toute autre valeur mathématique représentative le plus exactement possible du paramètre ou de son évolution sur une séance de dialyse.

Détermination du score de risque élevé (2):

Le système a les moyens programmés pour déterminer un score élevé comportant au moins un parmi les moyens suivants :

- des moyens programmés pour déterminer un premier critère de score élevé,
- des moyens programmés pour déterminer un deuxième critère de score élevé,
- des moyens programmés pour déterminer un troisième critère de score élevé,
- des moyens programmés pour déterminer un quatrième critère de score élevé,
- des moyens programmés pour déterminer un cinquième critère de score élevé,

et où les moyens programmés pour déterminer si le score est élevé sont capables d'émettre pour résultat :

- un score élevé lorsqu'au moins un parmi cinq critères de score de risque élevé est vérifié,
- un score non élevé lorsque tous les cinq critères de score de risque élevé sont non vérifiés.

En effet, si un seul parmi les 5 critères de score élevé est rempli, ceci suffit à en déduire le score de risque élevé.

On pourra également prévoir un niveau de score de risque élevé en fonction du nombre de critères de score élevé remplis. Au plus un patient aura de critères de score élevé remplis, au plus la prise en charge de ce patient sera prioritaire. La priorité du score de risque élevé pourra également être calculée et retenue pour la présentation des résultats au médecin.

Critères de score :

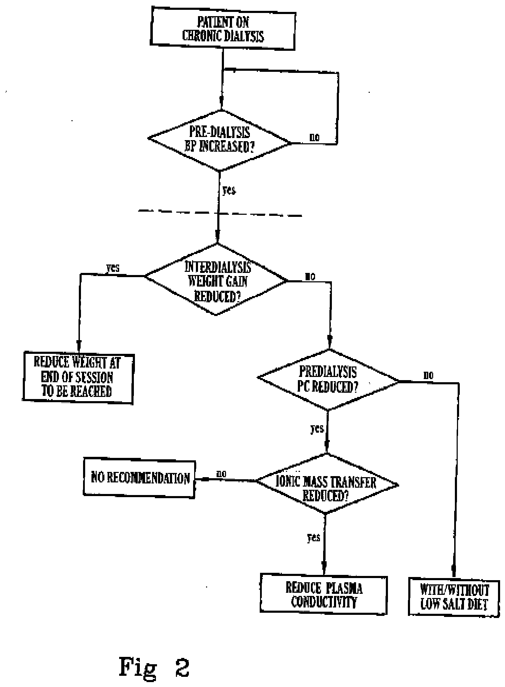

Il faut noter de façon générale que, malgré la schématisation étape après étape du diagramme des figures 2 et 3, la vérification des cinq critères de score élevé et des six critères de score nul peut être effectuée simultanément et sans imposition quant à l'ordre temporel des vérifications. La représentation graphique n'est faite que par souci de clarté.

Nous allons examiner en détails chacun des cinq critères de score de risque élevé.

Premier critère de score de risque élevé (2):

Il s'agit de déterminer si un premier critère de score de risque élevé est vérifié ou non.

Selon l'invention, le système a les moyens programmés pour déterminer un premier critère de score de risque élevé qui sont destinés à opérer au moins en fonction des paramètres d'efficacité (Pli, P1j, P2i, P2j, ...) et du débit sanguin extracorporel (Qbi,..., Qbj) déterminés pour au moins deux séances.

Plus particulièrement, les moyens programmés pour déterminer le premier critère de score de risque élevé peuvent être destinés au moins à comparer, pour au moins deux séances, chaque valeur du paramètre d'efficacité (Ei,..., Ej) d'une séance avec une fonction linéaire de la valeur du débit sanguin extracorporel (Qbi,..., Qbj) de la même séance.

Plus particulièrement, les moyens programmés pour déterminer le premier critère de risque élevé vérifié peuvent être destinés à déterminer pour au moins deux séances si chaque valeur de l'efficacité (E(i)) déterminée d'une séance se situe sur ou au-dessous la droite d'équation :

$$E(i) = 0,4 * Qb(i) + 40,$$

avec Qb(i) le débit sang du patient pour la même séance (i) ;
le score étant élevé si ce critère est vérifié.

En effet, l'équation de cette droite correspond aux valeurs exemplatives suivantes :

Pour Qsg=250mL/mn, l'efficacité devrait être égale ou supérieure à 140.
Pour Qsg= 300 mL/mn, l'efficacité devrait être égale ou supérieure à 160.
Pour Qsg= 350 mL/mn, l'efficacité devrait être égale ou supérieure à 180.
Pour: Qsg= 400 mL/mn, l'efficacité devrait être égale ou supérieure à 200.

De façon générale dans toute la présente demande, il faut remarquer qu'il s'agit de valeurs calculées pour les équations de droite données, mais que ces données comparées correspondent à des valeurs numériques, car les unités ne sont pas respectées.

Deuxième critère de score de risque élevé :

Il s'agit de déterminer si un deuxième critère de score élevé est vérifié ou non.

Selon l'invention, le système a les moyens programmés pour déterminer un deuxième critère de score de risque élevé qui peuvent être destinés à opérer au moins en fonction des valeurs de la pression veineuse (Pvi,..., Pvj) et de la pression artérielle (Pai,...,Paj) déterminées pour au moins deux séances.

Plus particulièrement, les moyens programmés pour déterminer le deuxième critère de score de risque élevé peuvent être destinés à comparer, pour au moins deux séances, la valeur de pression artérielle et respectivement la valeur de pression veineuse d'une séance avec une valeur de pression artérielle prédéterminée et respectivement une valeur de pression veineuse prédéterminée.

Plus particulièrement, le système a les moyens programmés pour déterminer le deuxième critère de score de risque élevé peuvent être destinés à déterminer pour au moins deux séances (i, j) si :

- la valeur de la pression veineuse (Pv(i), ...,Pv(j)) est supérieure ou égale à 250mmHg, et
- la valeur de la pression artérielle (Pa(i), ...,Pa(j)) est inférieure à -200mmHg,

le score étant élevé si ces deux conditions sont vérifiées. Les valeurs de seuil peuvent bien sûr varier dans un intervalle autour des valeurs indiquées, en fonction du patient, par exemple. Une valeur seuil de pression veineuse peut être comprise entre 200 et 300, environ 250 préférentiellement.

Au plus le nombre de séances pour lequel ce premier critère examiné est vérifié, au plus le résultat obtenu sur ce critère est sur.

Troisième critère de score de risque élevé :

Il s'agit de déterminer si un troisième critère de score élevé est vérifié ou non.

Selon l'invention, le système a les moyens programmés pour déterminer un troisième critère de score de risque élevé qui peuvent être destinés à opérer au moins en fonction de l'évolution des valeurs de pression veineuse (Pv(j)-Pv(i)), de l'évolution des valeurs de pression artérielle (Pa(j)-Pa(i)) et de l'évolution des valeurs d'efficacité (E(j)-E(i)) déterminées entre *une séance antérieure* (i) *et une séance postérieure (j).*

Plus particulièrement, le système a les moyens programmés pour déterminer le troisième critère de score de risque élevé qui peuvent être destinés à:

- comparer l'évolution de l'efficacité (E(j)-E(i)) relativement à la valeur de l'efficacité de la séance antérieure (E(i)), et

- comparer l'évolution des pressions artérielle (Pa(j)-Pa(i)) et veineuse (Pv(j)-Pv(i)) à une valeur prédéterminée.

Plus particulièrement, le système a les moyens programmés pour déterminer le troisième critère de score de risque élevé qui peuvent être destinés à déterminer si :

- la valeur absolue de la variation de l'efficacité (E(j)-E(i)) entre *la séance antérieure* (i) *et la séance postérieure (j)* est supérieure ou égale à 10%, préférentiellement 20%, de la valeur de l'efficacité de la séance antérieure (E(i)), et
- l'augmentation de la pression veineuse (Pv(j)-Pv(i)) entre *la séance antérieure* (i) *et la séance postérieure (j)* est supérieure ou égale à 50 mmHg, et
- la diminution de la pression artérielle (Pa(j)-Pa(i)) entre *la séance antérieure* (i) *et la séance postérieure (j)* est inférieure ou égale à 50 mmHg,

le score étant élevé si ces trois conditions sont vérifiées.
La valeur seuil de 50 mmHg est une valeur indicative, mais le seuil peut varier et être fixé à partir de 40 mmHg. Cette valeur peut être choisie par le médecin en fonction de la pression artérielle ou veineuse habituelle du patient. Il est de même pour le pourcentage seuil de la variation de l'efficacité.

Quatrième critère de score de risque élevé :

Il s'agit de déterminer si un quatrième critère de score élevé est vérifié ou non.
Selon l'invention, le système a les moyens programmés pour déterminer un quatrième critère de score de risque élevé qui peuvent être destinés à opérer au moins en fonction de l'évolution des valeurs de pression veineuse (Pv(j)-Pv(i)), de l'évolution des valeurs de pression artérielle (Pa(j)-Pa(i)) et de l'évolution des valeurs d'efficacité (E(j)-E(i)) déterminées entre une séance antérieure (i) et une séance postérieure (j).
Plus particulièrement, le système a les moyens programmés pour déterminer le quatrième critère de score de risque élevé qui peuvent être destinés à comparer chacune desdites trois évolutions de paramètres relativement à la valeur du paramètre de la séance antérieure.
Plus particulièrement, le système a les moyens programmés pour déterminer le quatrième critère de score de risque élevé qui peuvent être destinés à déterminer si :

- l'augmentation de la pression veineuse (Pv(j)-Pv(i)) entre la séance antérieure (i) et la séance postérieure (j) est supérieure ou égale à 10%, préférentiellement 20% de la valeur de la pression veineuse de la séance antérieure (Pv(i)), et

- la diminution de la pression artérielle (Pa(j)-Pa(i)) entre lesdites séances est supérieure ou égale à 10%, préférentiellement 20% de la valeur de la pression artérielle (Pv(i)) de la séance antérieure,

le score étant élevé si ces deux conditions sont vérifiées.
La valeur pourcentage seuil donnée est une valeur indicative, mais le seuil peut varier et être fixé entre 10% et 20%. Cette valeur peut être choisie par le médecin en fonction de la pression artérielle ou veineuse habituelle du patient.

Cinquième critère de score de risque élevé :

Il s'agit de déterminer si un cinquième critère de score élevé est vérifié ou non.
Le système selon l'invention a les moyens programmés pour déterminer le cinquième critère de score de risque élevé qui peuvent être destinés à opérer au moins en fonction de l'évolution de l'efficacité d'épuration (E(i)-E(j)) *entre une séance antérieure* (i) *et une séance postérieure (j).* Plus particulièrement, le système a les moyens programmés pour déterminer le cinquième critère de score de risque élevé qui peuvent être destinés à comparer l'évolution de l'efficacité d'épuration (E(i)-E(j)) avec une valeur prédéterminée.
Plus particulièrement, le système a les moyens programmés pour déterminer le cinquième critère de score de risque élevé qui peuvent être destinés à déterminer si la diminution de l'efficacité d'épuration (E(i)-E(j)) entre la séance antérieure (i) et la séance postérieure (j) est supérieure ou égale à 40mL/mn, le score étant élevé si cette condition est vérifiée.
La valeur seuil de 40 mL/mn est une valeur indicative, mais le seuil peut varier et être fixé à partir de 30 mL/mn. Cette valeur peut être choisie par le médecin en fonction de l'efficacité d'épuration habituelle sur le patient.
Par ailleurs, selon l'invention :

- pour la détermination du premier critère de score de risque élevé, si le nombre de séances considérées est supérieur à deux, alors les moyens de détermination correspondant sont destinés à opérer avec chaque séance considérée ;
- pour la détermination du deuxième critère de score de risque élevé, si le nombre de séances considérées est supérieur à deux, alors les moyens de détermination correspondant sont destinés à opérer avec au moins deux séances successives (i,i+1) ;
- pour la détermination d'au moins un parmi les troisième, quatrième et cinquième critères de score de risque élevé, si le nombre de séances considérées est supérieur à deux, alors les moyens de détermination correspondant sont destinés à opérer en identifiant la première séance temporelle considérée comme séance antérieure et en identifiant la dernière séance temporelle considérée comme séance postérieure ;

- pour la détermination d'au moins un parmi les troisième, quatrième et cinquième critères de score de risque élevé, si le nombre de séances considérées est supérieur à deux, alors les moyens de détermination correspondant sont destinés à considérer comme séances temporelles celles pour lesquelles les valeurs d'au moins un paramètre considéré sont les plus éloignées.

Il peut être néanmoins aussi valable de considérer plus de deux séances afin de discerner l'évolution au fil des séances.

Détermination score de risque nul (0) :

Le système comprend des moyens programmés pour déterminer le score de risque nul comprenant:

- des moyens programmés pour déterminer un premier critère de score nul,
- des moyens programmés pour déterminer un deuxième critère de score nul,
- des moyens programmés pour déterminer un troisième critère de score nul,
- des moyens programmés pour déterminer un quatrième critère de score nul,
- des moyens programmés pour déterminer un cinquième critère de score nul,
- des moyens programmés pour déterminer un sixième critère de score nul,
- et où les moyens programmés pour déterminer si le score est nul sont capables d'émettre pour résultat :
- un score nul lorsque six critères de score de risque nul sont tous vérifiés, ou
- un score non nul lorsqu'au moins un parmi les six critères de score nul n'est pas vérifié.

Premier critère de score de risque nul:

Il s'agit de déterminer si un premier critère de score de risque nul est vérifié ou non.

Les moyens programmés pour déterminer un premier critère de score de risque nul peuvent être destinés à opérer au moins en fonction du paramètre d'efficacité (E(i),...,E(j)) et du débit sanguin extracorporel (Qb(i), ...,Qb(j)) déterminés pour au moins deux séances.

Plus particulièrement, les moyens programmés pour déterminer le premier critère de score de risque nul peuvent être destinés à comparer, pour au moins deux séances, chaque valeur du paramètre d'efficacité (E(i),...,E(j)) d'une séance avec une fonction linéaire de la valeur du débit sanguin extracorporel (Qb(i),..., Qb(j)) de la même séance.

Plus particulièrement, les moyens programmés pour déterminer le premier critère de risque nul peuvent être destinés à déterminer, pour au moins deux séances, si chaque valeur de l'efficacité déterminée d'une séance se situe sur ou au-dessus la droite d'équation :

$$E(i) = 0,4 * Qb(i) + 40,$$

avec Qb(i) le débit sang extracorporel pour la même séance (i), le premier critère de score de risque nul étant vérifié dans ce cas.

Au plus le nombre de séances pour lequel ce premier (ou tout autre) critère examiné est vérifié, au plus le résultat obtenu sur ce critère est sûr.

En effet, l'équation de cette droite correspond aux valeurs exemplatives suivantes :

Pour Qb= 250mL/mn, l'efficacité devrait être égale ou supérieure à 140.
Pour Qb= 300 mL/mn, l'efficacité devrait être égale ou supérieure à 160.
Pour Qb= 350 mL/mn, l'efficacité devrait être égale ou supérieure à 180.
Pour: Qb= 400 mL/mn, l'efficacité devrait être égale ou supérieure à 200.

Deuxième critère de score de risque nul :

Il s'agit de déterminer si un deuxième critère de score de risque nul est vérifié ou non.

Le système comprend des moyens programmés pour déterminer un deuxième critère de score de risque nul qui sont destinés à opérer au moins en fonction des valeurs de la pression veineuse (Pv(i),..., Pv(j)) et de la pression artérielle (Pa(i),..., Pa(j)) et des valeurs du débit sanguin du patient (Qb(i),..., Qb(j)) déterminées pour au moins deux séances.

Plus particulièrement, les moyens programmés pour déterminer un deuxième critère de score de risque nul peuvent être destinés à comparer, pour au moins deux séances, chaque valeur de la pression artérielle (Pa(i),... ,Pa(j)) avec une fonction linéaire du débit sanguin (Qb(i),...,Qb(j)) de la séance et chaque valeur de la valeur de pression veineuse (Pv(i),...,Pv(j)) avec une fonction linéaire du débit sanguin de la séance (Qb(i),...,Qb(j)).

Plus particulièrement, les moyens programmés pour déterminer le deuxième critère de score de risque nul peuvent être destinés à déterminer pour au moins deux séances (i, j) :

- si chaque valeur absolue de la pression artérielle (Pa(i),..., Pa(j)) déterminée par séance est inférieure ou égale à la moitié du débit sanguin du patient (Qb(i), ...,Qb(j)) pour la séance considérée (i,...,j), et
- si chaque valeur de la pression veineuse (Pv(i), ...,Pv(j)) déterminée par séance est inférieure ou égale à la moitié du débit sanguin du patient (Qb(i),..., Qb(j)) pour la séance considérée (i,...,j),

le deuxième critère de score de risque nul étant vérifié dans ce cas.

En effet, l'équation de cette droite correspond aux valeurs exemplatives suivantes :

Pour Qb= 250mL/mn, la pression veineuse devrait être inférieure ou égale à 125mmHg et la pression artérielle devrait être supérieure ou égale à -125mmHg

Pour Qb= 300 mL/mn, la pression veineuse devrait être inférieure ou égale à 150mmHg et la pression artérielle devrait être supérieure ou égale à -150mmHg

Pour Qb= 350 mL/mn, la pression veineuse devrait être inférieure ou égale à 175 mmHg et la pression artérielle devrait être supérieure ou égale à -175mmHg

Pour Qb= 400 mL/mn, la pression veineuse devrait être inférieure ou égale à 200 mmHg et la pression artérielle devrait être supérieure ou égale à -200mmHg)

Troisième critère de score de risque nul:

Il s'agit de déterminer si un troisième critère de score de risque nul est vérifié ou non.

Le système comprend des moyens programmés pour déterminer le troisième critère de score de risque nul qui peuvent être destinés à opérer au moins en fonction de l'évolution du débit sang (Qb(j)- Qb(i)) extracorporel entre *une séance antérieure* (i) *et une séance postérieure (j).*

Plus particulièrement, les moyens programmés pour déterminer le troisième critère de score de risque nul peuvent être destinés à comparer l'évolution du débit sanguin du patient (Qb(j)- Qb(i)) à une valeur prédéterminée.

Plus particulièrement, les moyens programmés pour déterminer le troisième critère de score de risque nul peuvent être destinés à déterminer si la valeur absolue de la variation du débit sang (|Qb(j)-Qb(i)|) entre une séance antérieure (i) et une séance postérieure (j) est inférieure ou égale à 20mL/mn, le troisième critère de score de risque nul étant vérifié dans ce cas.

La valeur de différence seuil de 20 mL/mn est une valeur indicative, mais le seuil peut varier et être fixé à partir de 10 mL/mn. Cette valeur peut âtre choisie par le médecin en fonction du débit sanguin habituel du patient en traitement, et varierait entre 10 et 20, voire supérieure à 20.

Quatrième critère de score de risque nul:

Il s'agit de déterminer si un quatrième critère de score de risque nul est vérifié ou non.

Le système comprend des moyens programmés pour déterminer le quatrième critère de score de risque nul qui peuvent être destinés à opérer au moins en fonction de l'évolution des valeurs de pression artérielle (Pa(j)-Pa(i)) entre *une séance antérieure* (i) *et une séance postérieure (j).*

Plus particulièrement, les moyens programmés pour déterminer le quatrième critère de score de risque nul peuvent être destinés à comparer l'évolution de la pression artérielle (Pa(j)-Pa(i)) à la valeur de la pression artérielle de la séance antérieure (Pa(i)).

Plus particulièrement, les moyens programmés pour déterminer le quatrième critère de score de risque nul peuvent être destinés à déterminer si la variation de la pression artérielle (Pa(j)-Pa(i)) entre la séance antérieure (i) et la séance postérieure (j) est inférieure ou égale à 10%, préférentiellement à 20%, de la pression artérielle (P(i)) de la séance antérieure, le quatrième critère de score de risque nul étant vérifié dans ce cas. La valeur pourcentage seuil donnée est une valeur indicative, mais le seuil peut varier et être fixé entre 10% et 20%. Cette valeur peut âtre choisie par le médecin en fonction de la pression artérielle habituelle du patient.

Cinquième critère de score de risque nul:

Il s'agit de déterminer si un cinquième critère de score de risque nul est vérifié ou non.

Le système comprend des moyens programmés pour déterminer le cinquième critère de score de risque nul qui peuvent être destinés à opérer au moins en fonction de l'évolution des valeurs de pression veineuse ((Pv(j)-Pv(i)) entre une séance antérieure et une séance postérieure.

Plus particulièrement, les moyens programmés pour déterminer le cinquième critère de score de risque nul peuvent être destinés à comparer l'évolution de la pression veineuse ((*Pv(j)*-*Pv(i)*) à la valeur de la pression veineuse de la séance antérieure (Pv(i)).

Plus particulièrement, les moyens programmés pour déterminer le cinquième critère de score de risque nul peuvent être destinés à déterminer si la variation de la pression veineuse ((*Pv(j)*-*Pv(i)*) entre la séance antérieure (i) et la séance postérieure (j) est inférieure ou égale à 10%, préférentiellement à 20%, de la pression veineuse (Pv(i)) de la séance antérieure, le cinquième critère de score de risque nul étant vérifié dans ce cas.

La valeur pourcentage seuil donnée est une valeur indicative, mais le seuil peut varier et être fixé entre 10% et 20%. Cette valeur peut âtre choisie par le médecin en fonction de la pression veineuse habituelle du patient.

Sixième critère de score de risque nul:

Il s'agit de déterminer si un sixième critère de score de risque nul est vérifié ou non.

Le système comporte des moyens programmés pour déterminer le sixième critère de score de risque nul qui peuvent être destinés à opérer au moins en fonction de l'évolution de l'efficacité du traitement ((E(j)-*E(i)*) entre *une séance antérieure* (i) *et une séance postérieure (j).*

Plus particulièrement, les moyens programmés pour déterminer le sixième critère de score de risque nul peuvent être destinés à comparer l'évolution de l'efficacité ((E(j)-*E(i))* du traitement à une valeur prédéterminée.

Plus particulièrement, les moyens programmés pour déterminer un sixième critère de score de risque nul peuvent être destinés à déterminer si la valeur absolue de la variation de l'efficacité du traitement ((E(*j*)-*E(i))* entre la séance antérieure (i) et la séance postérieure (j) est inférieure ou égale à 10mL/mn, le sixième critère de score de risque nul étant vérifié dans ce cas.

La valeur seuil de 10 mL/mn est une valeur indicative, mais le seuil peut varier et être fixé à partir de 5 mL/mn. Cette valeur peut âtre choisie par le médecin en fonction de l'efficacité d'épuration habituelle sur le patient.

Dans le système :

- pour la détermination d'au moins un parmi le premier et le deuxième critères de score de risque nul, si le nombre de séances considérées est supérieur à deux, alors les moyens de détermination correspondant sont destinés à opérer avec chaque séance considérée ;

- pour la détermination d'au moins un parmi le troisième, quatrième, cinquième et sixième critères de score de risque nul, si le nombre de séances considérées est supérieur à deux, alors les moyens de détermination correspondant sont destinés à opérer en identifiant la première séance temporelle considérée comme séance antérieure et en identifiant la dernière séance temporelle considérée comme séance postérieure ;

- pour la détermination d'au moins un parmi les troisième, quatrième, cinquième et sixième critères de score de risque nul, si le nombre de séances considérées est supérieur à deux, alors les moyens de détermination correspondant sont destinés à considérer comme séances temporelles celles pour lesquelles les valeurs d'au moins un paramètre considéré sont les plus éloignées.

Dans le système, les séances de traitement considérées s'étalent sur au moins deux semaines, préférentiellement sur un intervalle compris entre deux semaines et six mois, plus préférentiellement sur un intervalle de trois semaines.

Système de détermination de fiabilité de score de risque

Un système de détermination de fiabilité de score de risque de l'état d'un accès vasculaire comprend des moyens suivant:

- le système de détermination de l'état d'un accès vasculaire décrit ci-dessus,
- des moyens pour mémoriser :

    I. un premier score de risque (S) déterminé sur un premier intervalle temporel défini entre une séance antérieure et une séance postérieure et comprenant plus de deux séances,

    II. au moins un deuxième score de risque (S') déterminé sur au moins un deuxième intervalle temporel situé à l'intérieur du premier intervalle déterminé,

- des moyens programmés pour calculer la fiabilité en fonction du premier score et au moins du deuxième score déterminés (S, S').

Plus particulièrement, lorsqu'un nombre n de scores de risque est déterminé par le système de détermination de l'état d'un accès vasculaire, les moyens programmés pour calculer la fiabilité peuvent être destinés à calculer le pourcentage de fiabilité comme égal ou lié au rapport du nombre d'identité(s) entre le premier score de risque déterminé sur le premier intervalle et chacun des autres scores de risque déterminés sur un intervalle à l'intérieur du premier intervalle divisé sur le nombre n.

Particulièrement, le deuxième intervalle temporel peut avoir pour borne postérieure la séance postérieure du premier intervalle, ou une borne très proche temporellement de la borne postérieure la séance postérieure du premier intervalle.

Il faut bien noter que le système selon l'invention n'est pas mis nécessairement en oeuvre pendant le traitement. Préférentiellement, il est mis en oeuvre après une séance de traitement, si les paramètres considérés sont des paramètres moyens sur une séance.

L'invention concerne aussi un ordinateur comprenant :

- des moyens de mémorisation stockant au moins des valeurs d'au moins un paramètre hémodynamique extracorporel (Pli, ..., P1j,...P2i,...,P2j...) et des valeurs d'efficacité d'épuration (E (i),...,E(j)) relatives à au moins un patient soumis à plusieurs séances (i,...j) de traitement extracorporel sanguin,
- un système de calcul et de commande selon l'invention pour la détermination de l'état vasculaire du patient dont les valeurs de paramètres d'au moins un paramètre hémodynamique extracorporel (Pli, ..., P1j,...P2i,...,P2j...) et des valeurs d'efficacité d'épuration (E(i),...,E(j)) sont stockées dans lesdits moyens de mémorisation.

L'invention concerne aussi une machine de traitement extracorporel de sang comprenant au moins:

- une unité de traitement de sang capable de mettre en oeuvre un traitement extracorporel sanguin par circulation de sang via un circuit extracorporel sanguin comprenant une ligne artérielle, une première chambre d'un filtre séparé par une membrane semi-perméable, une ligne veineuse et par circulation de dialysat dans une deuxième chambre du filtre,

- des moyens de mémorisation stockant au moins des valeurs d'au moins un paramètre hémodynamique extracorporel et des valeurs d'efficacité d'épuration relatives à au moins un patient soumis à plusieurs séances de traitement extracorporel sanguin,
- un système de calcul et de commande selon l'invention pour la détermination de l'état vasculaire du patient dont les valeurs de paramètres d'au moins un paramètre hémodynamique extracorporel (Pli, ..., P1j,...P2i,...,P2j...) et des valeurs d'efficacité d'épuration (E(i),...,E(j)) sont stockées dans lesdits moyens de mémorisation.

L'invention concerne aussi un réseau comprenant:

- un serveur,
- au moins une machine de traitement de sang reliée au serveur, chaque machine comprenant :

  - des moyens de mesure et/ou de calcul de données médicales relatives à au moins un paramètre hémodynamique extracorporel (P1i,..., P1j,...P2i,...,P2j...) et à l'efficacité d'épuration du traitement (E (i),...,E(j)),
  - des moyens d'émission d'au moins une partie de ces données mesurées et/ou calculées vers le serveur,

- le serveur comprenant :

  - des moyens de réception d'au moins une partie des données médicales relatives à des traitements extracorporels de sang,
  - des moyens de mémorisation pour stocker les données reçues par les moyens de réception depuis une ou plusieurs machines de traitement de sang,
  - un système de calcul et de commande selon l'invention, destiné à opérer à partir desdites données reçues,

- au moins un poste (poste client par exemple) capable de communiquer avec le serveur pour recevoir au moins les résultats de la mise en oeuvre dudit système de calcul et de commande.

Le poste peut comprendre une unité d'affichage des résultats de score de risque.

Ce procédé est décrit pendant le traitement, mais peut être mis en oeuvre après le traitement et sur un lieu éloigné pour l'étape de sélection (comme sur la figure 15).

Une fois les paramètres nécessaires sélectionnés, le serveur met en oeuvre le procédé selon l'invention, ce procédé étant automatisé par la mise en oeuvre d'un logiciel expert.

L'utilisateur pourra accéder au moins aux résultats du procédé via un poste relié audit serveur.

Les liaisons décrites peuvent être sécurisées par des techniques connues pour des raisons de confidentialité des données relatives à un patient. Alternativement ou additionnellement, les données peuvent être, avant émission, « anonymisées » par l'attribution d'un code à chaque patient, sans faire apparaître le nom du patient lors des échanges de données.

Encore, l'invention concerne un procédé de détermination de l'état d'un accès vasculaire d'un patient destiné à suivre des séances successives (i,j) de traitement extracorporel de sang par extraction et retour du sang via l'accès vasculaire, le procédé de détermination comprenant les étapes suivantes:

a) déterminer, la valeur (Pi, Pj) d'au moins un paramètre (P) extracorporel hémodynamique du patient pour au moins deux séances (i,j),
b) déterminer la valeur (Ei, Ej) de l'efficacité d'épuration du traitement pour au moins deux séances (i,j),
c) déterminer un score de risque concernant l'état de l'accès vasculaire du patient en fonction desdites au moins deux valeurs (Pi, Pj) du paramètre extracorporel hémodynamique et desdites au moins deux valeurs (Ei, Ej) de l'efficacité d'épuration déterminées.

Ce procédé préférentiellement mis en oeuvre automatiquement peut être conduit sur place dans la salle de traitement ou à distance dans une salle de la clinique ou d'un centre de traitement de données.

Dans le procédé, le score de risque peut prendre trois valeurs :

s0) score de risque nul pour un patient dont l'état de l'accès vasculaire est normal,
s1) score de risque intermédiaire pour un patient dont l'état de l'accès vasculaire est mitigé (ou « douteux »),
s2) score de risque élevé pour un patient dont l'état de l'accès vasculaire est alarmant.

La détermination du score de risque peut comporter :

- l'étape de déterminer si le score de risque est élevé,
- l'étape additionnelle de déterminer si le score de risque est nul,
- l'étape additionnelle suivante: si le score de risque n'est déterminé ni alarmant ni nul, alors il est considéré intermédiaire.

L'étape de déterminer si le score de risque est nul peut être effectuée après l'étape de déterminer si le score de risque est élevé.

Paramètres en jeu:

Dans le procédé, le ou les paramètres extracorporels hémodynamiques (P) ont été mesurés pour une séance de traitement extracorporel sanguin qui consiste à circuler le sang du patient à un débit extracorporel sanguin et dans un circuit extracorporel sanguin, ce circuit comprenant une ligne artérielle où existe une pression artérielle, un filtre et une ligne veineuse où existe une pression veineuse.

Ces paramètres peuvent être choisis parmi le groupe comprenant:

- Pression veineuse (Pv) extracorporelle,
- Pression artérielle (Pa) extracorporelle,
- débit sang extracorporel du patient(Qb),
- un paramètre proportionnel à un des trois précités paramètres.

Dans le procédé, l'efficacité d'épuration (E) a été mesurée pour une séance de traitement extracorporel sanguin qui consiste à circuler le sang du patient à un débit extracorporel sanguin et dans un circuit extracorporel sanguin, ce circuit comprenant une ligne artérielle extracorporelle, un filtre et une ligne veineuse extracorporelle, l'efficacité d'épuration (E) étant égale ou fonction d'au moins un des paramètres suivants :

- la dialysance (D),ou
- la clairance (C), ou
- la concentration d'une substance contenue dans le sang avant le filtre (Cbin), ou
- la concentration d'une substance contenue dans le sang après le filtre (Cbout) dans le circuit extracorporel, ou
- la dose de dialyse (KT/v), ou
- un paramètre proportionnel à un des cinq précités paramètres.

Les valeurs du ou des paramètres hémodynamiques et de l'efficacité d'épuration peuvent des valeurs moyennes de ces paramètres sur une séance de traitement.

Détermination du score de risque élevé (2):

Le procédé comporte la détermination si le score est élevé a :

- pour résultat un score élevé lorsqu'au moins un parmi cinq critères de score de risque élevé est vérifié,
- pour résultat un score non élevé lorsque tous les cinq critères de score de risque élevé sont non vérifiés.

Détermination du premier critère de score de risque élevé :

La détermination d'un premier critère de score de risque élevé est au moins fonction du paramètre d'efficacité et du débit sanguin extracorporel déterminés pour au moins deux séances.

Plus particulièrement, la détermination du premier critère de score de risque élevé est effectuée au moins en comparant, pour au moins deux séances, chaque valeur du paramètre d'efficacité d'une séance avec une fonction linéaire de la valeur du débit sanguin extracorporel de la même séance.

Plus particulièrement, la détermination du premier critère de risque élevé vérifié consiste à déterminer pour au moins deux séances si chaque valeur de l'efficacité déterminée d'une séance se situe sur ou au-dessous la droite d'équation :

$$E(i) = 0,4 * Qb(i) + 40,$$

avec Qb(i) le débit sang du patient pour la même séance (i) ; le score étant élevé si ce critère est vérifié.

Au plus le nombre de séances pour lequel ce premier critère examiné est vérifié, au plus le résultat obtenu sur ce critère est sûr.

Deuxième critère de score de risque élevé :

La détermination d'un deuxième critère de score de risque élevé est au moins fonction des valeurs de la pression veineuse et de la pression artérielle déterminées pour au moins deux séances.

Plus particulièrement, la détermination du deuxième critère de score de risque élevé est faite en comparant, pour au moins deux séances, la valeur de pression artérielle et respectivement la valeur de pression veineuse d'une séance avec une valeur de pression artérielle prédéterminée et respectivement une valeur de pression veineuse prédéterminée.

Plus particulièrement, la détermination du deuxième critère de score de risque élevé vérifié consiste à déterminer pour au moins deux séances (i, j) si :

- la valeur de la pression veineuse (Pv(i), Pv(j)) est supérieure ou égale à 250mmHg, et
- la valeur de la pression artérielle (Pa(i), Pa(j)) est inférieure à -200mmHg pour au moins deux séances (i,j);

le score étant élevé si ces deux conditions sont vérifiées.

Troisième critère de score de risque élevé:

La détermination d'un troisième critère de score de risque élevé est au moins en fonction de l'évolution des valeurs de pression veineuse, de l'évolution des valeurs de pression artérielle et de l'évolution des valeurs d'efficacité déterminées entre *une séance antérieure* (i) *et une séance postérieure (j).*

Plus particulièrement, la détermination du troisième critère de score de risque élevé comporte:

- la comparaison de l'évolution de l'efficacité (E(j)-E(i)) relativement à la valeur de l'efficacité de la séance antérieure (E(i)), et
- la comparaison de l'évolution des pressions artérielle (Pa(j)-Pa(i)) et veineuse (Pv(j)-Pv(i)) à une valeur prédéterminée.

Plus particulièrement, la détermination du troisième critère de score de risque élevé consiste à déterminer si :

- la valeur absolue de la variation de l'efficacité (E(j)-E(i)) entre *la séance antérieure* (i) *et la séance postérieure (j)* est supérieure ou égale à 10%, préférentiellement 20% de la valeur de l'efficacité de la séance antérieure (E(i)), et
- l'augmentation de la pression veineuse (Pv(j)-Pv(i)) entre *la séance antérieure* (i) et *la séance postérieure (j)* est supérieure ou égale à 50mmHg, et
- la diminution de la pression artérielle (Pa(j)-Pa(i)) entre *la séance antérieure* (i) *et la séance postérieure (j)* est inférieure ou égale à 50 mmHg,

le score étant élevé si ces trois conditions sont vérifiées.

Quatrième critère de score de risque élevé:

La détermination d'un quatrième critère de score de risque élevé est au moins fonction de l'évolution des valeurs de pression veineuse (Pv(j)-Pv(i)), de l'évolution des valeurs de pression artérielle (Pa(j)-Pa(i)) et de l'évolution des valeurs d'efficacité (E(j)-E(i)) déterminées entre *une séance antérieure* (i) *et une séance postérieure (j).*

Plus particulièrement, la détermination du quatrième critère de score de risque élevé comporte la comparaison de chacune desdites trois évolutions de paramètres relativement à la valeur du paramètre de la séance antérieure.

Plus particulièrement, la détermination du quatrième critère de score de risque élevé consiste à déterminer si :

- l'augmentation de la pression veineuse (Pv(j)-Pv(i)) entre *la séance antérieure* (i) *et la séance postérieure (j)* est supérieure ou égale à 10%, préférentiellement 20% de la valeur de la pression veineuse de la séance antérieure (Pv(i)), et
- la diminution de la pression artérielle (Pa(j)-Pa(i)) entre lesdites séances est supérieure ou égale à 10%, préférentiellement 20% de la valeur de la pression artérielle (Pv(i)) de la séance antérieure,

le score étant élevé si ces deux conditions sont vérifiées.

Cinquième critère de score de risque élevé:

La détermination d'un cinquième critère de score de risque élevé est au moins fonction de l'évolution de l'efficacité d'épuration (E(i)-E(j)) *entre une séance antérieure* (i) *et une séance postérieure (j).*

Plus particulièrement, la détermination du cinquième critère de score de risque élevé comporte la comparaison de l'évolution de l'efficacité d'épuration (E(i)-E(j)) avec une valeur prédéterminée.

Plus particulièrement, la détermination du cinquième critère de score de risque élevé vérifié consiste à déterminer si la diminution de l'efficacité d'épuration (E(i)-E(j)) *entre la séance antérieure* (i) *et la séance postérieure (j)* est supérieure ou égale à 40mL/mn, le score étant élevé si cette condition est vérifiée.

Selon un exemple :

- pour la détermination du premier critère de score de risque élevé, si le nombre de séances considérées est supérieur à deux, alors la détermination peut être faite pour chaque séance considérée.
- pour la détermination du deuxième critère de score de risque élevé, si le nombre de séances considérées est supérieur à deux, alors la détermination peut être faite pour au moins deux séances successives (i,i+1).
- pour la détermination d'au moins un parmi les troisième, quatrième et cinquième critères de score de risque élevé, si le nombre de séances considérées est supérieur à deux, alors la séance antérieure peut être la première séance temporelle considérée et la séance postérieure peut être la dernière séance temporelle considérée,

- pour la détermination d'au moins un parmi les troisième, quatrième et cinquième critères de score de risque élevé, si le nombre de séances considérées est supérieur à deux, alors les séances temporelles considérées peuvent être celles pour lesquelles les valeurs d'au moins un paramètre considéré sont les plus éloignées.

Détermination score de risque nul (0):

Cette détermination a pour résultat:

- un score nul lorsque six critères de score de risque nul sont tous remplis, ou
- un score non nul lorsqu'au moins un parmi les six critères de score nul n'est pas rempli.

Premier critère de score de risque nul:

La détermination d'un premier critère de score de risque nul est au moins fonction du paramètre d'efficacité et du débit sanguin extracorporel déterminés pour au moins deux séances.

Plus particulièrement, la détermination du premier critère de score de risque nul est effectuée en comparant, pour au moins deux séances, chaque valeur du paramètre d'efficacité d'une séance avec une fonction linéaire de la valeur du débit sanguin extracorporel de la même séance.

Plus particulièrement, la détermination du premier critère de risque nul consiste à déterminer, pour au moins deux séances, si chaque valeur de l'efficacité déterminée d'une séance se situe sur ou au-dessus de la droite d'équation :

$$E(i) = 0,4 * Qb(i) + 40,$$

avec Qb(i) le débit sang extracorporel pour la même séance (i), le premier critère de score de risque nul étant vérifié dans ce cas.

Deuxième critère de score de risque nul :

La détermination d'un deuxième critère de score de risque nul est au moins fonction des valeurs de la pression veineuse et de la pression artérielle et des valeurs du débit sanguin du patient déterminées pour au moins deux séances.

Plus particulièrement, la détermination du deuxième critère de score de risque nul est faite en comparant, pour au moins deux séances, chaque valeur de la pression artérielle (Pa(i),...,Pa(j)) avec une fonction linéaire du débit sanguin (Qb(i),...,Qb(j)) de la séance et chaque valeur de la valeur de pression veineuse (Pv(i),...,Pv(j)) avec une fonction linéaire du débit sanguin de la séance (Qb(i),...,Qb(j)). Plus particulièrement, la détermination du deuxième critère de score de risque nul consiste à déterminer pour au moins deux séances (i, j) :

- si chaque valeur absolue de la pression artérielle (Pa(i), Pa(j)) déterminée par séance est inférieure ou égale à la moitié du débit sanguin du patient (Qb(i),..., Qb(j)) pour la séance considérée (i,j), et
- si chaque valeur de la pression veineuse (Pv(i),..., Pv(j)) déterminée par séance est inférieure ou égale à la moitié du débit sanguin du patient (Qb(i),..., Qb(j)) pour la séance considérée (i,j),

le deuxième critère de score de risque étant considéré nul dans ce cas.

Troisième critère de score de risque nul:

La détermination d'un troisième critère de score de risque nul est au moins en fonction de l'évolution du débit sang extracorporel (Qb(j)-Qb(i)) entre *une séance antérieure* (i) et *une séance postérieure (j)*.

Plus particulièrement, la détermination du troisième critère de score de risque nul comporte la comparaison de l'évolution du débit sanguin du patient à une valeur prédéterminée.

Plus particulièrement, la détermination du troisième critère de score de risque nul consiste à déterminer si la valeur absolue de la variation du débit sang (|Qb(j)-Qb(i)|) entre une séance antérieure (i) et une séance postérieure (j) est inférieure ou égale à 20mL/mn, le troisième critère de score de risque étant considéré nul dans ce cas.

Quatrième critère de score de risque nul:

La détermination d'un quatrième critère de score de risque nul est au moins fonction de l'évolution des valeurs de pression artérielle (Pa(j)-Pa(i)) entre *une séance antérieure* (i) *et une séance postérieure (j)*.

Plus particulièrement, la détermination du quatrième critère de score de risque nul comporte la comparaison de l'évolution de la pression artérielle (Pa(j)-Pa(i)) à la valeur de la pression artérielle (Pa(i)) de la séance antérieure (i).

Plus particulièrement, la détermination du quatrième critère de score de risque nul consiste à déterminer si la variation de la pression artérielle (Pa(j)-Pa(i)) entre la séance antérieure (i) et la séance postérieure (j) est inférieure ou égale à 10%, préférentiellement à 20%, de la pression artérielle (Pa(i)) de la séance antérieure, le quatrième critère de score de risque étant considéré nul dans ce cas.

Cinquième critère de score de risque nul:

La détermination d'un cinquième critère de score de risque nul est au moins fonction de l'évolution des valeurs de pression veineuse (Pv(j)-Pv(i)) entre *une séance antérieure* (i) *et une séance postérieure (j)*.

Plus particulièrement, la détermination du cinquième critère de score de risque nul comporte la comparaison de l'évolution de la pression veineuse (Pv(j)-Pv(i)) à la valeur de la pression veineuse de la séance antérieure (Pv(i)).

Plus particulièrement, la détermination du cinquième critère de score de risque nul consiste à déterminer si la variation de la pression veineuse (Pv(j)-Pv(i)) entre la séance antérieure (i) et la séance postérieure (j) est inférieure ou égale à 10%, préférentiellement à 20%, de la pression veineuse (Pv(i)) de la séance antérieure, le cinquième critère de score de risque étant considéré nul dans ce cas.

Sixième critère de score de risque nul:

La détermination d'un sixième critère de score de risque nul est au moins en fonction de l'évolution de l'efficacité du traitement ((E(j)-*E(i))* entre *une séance antérieure* (i) *et une séance postérieure (j).*

Plus particulièrement, la détermination du sixième critère de score de risque nul comporte la comparaison de l'évolution de l'efficacité du traitement ((E(j)-*E(i))* à une valeur prédéterminée.

Plus particulièrement, la détermination du sixième critère de score de risque nul consiste à déterminer si la valeur absolue de la variation de l'efficacité du traitement ((E(j)-*E(i))* entre la séance antérieure (i) et la séance postérieure (j) est inférieure ou égale à 10mL/mn, le sixième critère de score de risque étant considéré nul dans ce cas.

Dans le procédé de détermination du score de risque nul :

- pour la détermination d'au moins un parmi le premier et le deuxième critères de score de risque nul, si le nombre de séances considérées est supérieur à deux, alors la détermination est faite pour chaque séance considérée,
- pour la détermination d'au moins un parmi le troisième, quatrième, cinquième et sixième critères de score de risque nul, si le nombre de séances considérées est supérieur à deux, alors la séance antérieure peut être la première séance temporelle considérée et la séance postérieure peut être la dernière séance temporelle considérée,
- pour la détermination d'au moins un parmi les troisième, quatrième, cinquième et sixième critères de score de risque nul, si le nombre de séances considérées est supérieur à deux, alors les séances temporelles considérées peuvent être celles pour lesquelles les valeurs d'au moins un paramètre considéré sont les plus éloignées.

Dans le procédé, les séances de traitement considérées s'étalent sur au moins deux semaines, préférentiellement sur un intervalle compris entre 2 semaines et 6 mois, plus préférentiellement sur un intervalle de 3 semaines.

Un procédé de détermination de fiabilité de score de risque de l'état d'un accès vasculaire comprend les étapes suivantes:

- première mise en oeuvre du procédé de détermination de l'état d'un accès vasculaire sur un premier intervalle temporel défini entre une séance antérieure et une séance postérieure et comprenant plus de deux séances,
- prise en compte du premier score de risque déterminé,
- au moins une deuxième mise en oeuvre du procédé de détermination de l'état d'un accès vasculaire sur au moins un deuxième intervalle temporel situé à l'intérieur du premier intervalle déterminé,
- prise en compte du deuxième score de risque déterminé,
- calculer la fiabilité en fonction du premier score et au moins du deuxième score déterminés.

Plus particulièrement, lorsqu'un nombre n de scores de risque est déterminé par n mises en oeuvre du procédé de détermination de l'état d'un accès vasculaire, le calcul d'un pourcentage de fiabilité est opéré par le rapport du nombre d'identité(s) entre le premier score de risque déterminé sur le premier intervalle et chacun des autres scores de risque déterminés sur un intervalle à l'intérieur du premier intervalle divisé sur le nombre n.

Plus particulièrement, le deuxième intervalle temporel a pour borne postérieure la séance postérieure du premier intervalle.

Il faut bien noter que le procédé selon l'invention n'est pas mis nécessairement en oeuvre pendant le traitement. Préférentiellement, il est mis en oeuvre après une séance de traitement, si les paramètres considérés sont des paramètres moyens sur une séance.

Un programme d'ordinateur pour la détermination de l'état d'un accès vasculaire d'un patient chargeable dans la mémoire interne d'un ordinateur, comprenant des portions de code de programme d'ordinateur pour, lorsque le programme est exécuté par l'ordinateur, mettre en oeuvre le procédé de détermination de l'état de l'accès vasculaire et/ou le procédé de détermination de fiabilité de score de risque de l'état déterminé.

Ce programme peut être enregistré sur un support lisible dans un ordinateur, le support étant une mémoire de données optique et/ou magnétique ou un support de mémoire volatile.

En général, le score de risque prend 3 valeurs selon les informations fournies par les paramètres examinés.

Le score de risque nul correspond aux patients examinés qui sont stables dans une zone de normalité pendant une période de temps récente et suffisamment longue.

Le score de risque intermédiaire correspond aux patients examinés peuvent être en dehors de la zone de normalité, mais qui n'ont pas d'aggravation récente de leur accès vasculaire permettant de prévoir une complication à court terme.

Le score de risque élevé correspond aux patients examinés exposés à une complication à court terme qui menace la fonctionnalité de l'accès vasculaire.

Avantages de la détermination de suivi de l'accès vasculaire:

- rapidité de l'évaluation d'un risque de l'accès vasculaire,
- mis en oeuvre de l'invention sans matériel supplémentaire nécessaire ;
- temps de traitement ou d'intervention supplémentaire sur le patient épargnés ;

- coûts supplémentaires épargnés de mains d'oeuvre, d'appareils médicaux consommables, de matériel (usage du Doppler...)
- mis en oeuvre de l'invention sans manipulation supplémentaire pendant le traitement, et sans intervention pendant les séances de dialyse, donc sans produire de perturbations,
- niveaux d'alerte triés par priorité avec un score de risque élevé et un score de risque intermédiaire ;
- suivi à distance de plusieurs patients et/ou dans une ou plusieurs cliniques,
- suivi à distance par un médecin d'un malade dialyse à domicile
- anticipation de l'état à risque de l'accès vasculaire avant le début d'une séance de dialyse,
- classement selon plusieurs niveaux d'importance du risque de l'accès vasculaire,
- le médecin peut se voir envoyer directement les scores calculés.

Chez les patients de score nul, la solution évite des explorations et/oui des examens couteux répétés par analyse régulière (chaque semaine par exemple) et systématique,
Chez les patients de score intermédiaire, une situation plutôt normale est détectée, mais l'analyse oriente le médecin vers des examens complémentaires et/ou oriente le médecin à prescrire une mise à jour de la prescription pour le traitement extracorporel,
Chez les patients de score élevé, il existe un gain de temps dans l'indication d'exploration invasive, et donc il existe de meilleures chances de sauver un accès vasculaire et d'accéder à des possibilités de traitements performants en matière d'épuration extrarénale.

**Revendications**

1. Système de calcul et de commande pour déterminer l'appartenance d'un patient destiné à suivre plusieurs séances (i,j) de traitement extracorporel de sang par extraction et retour du sang via l'accès vasculaire, à un groupe parmi plusieurs groupes de patients hypertendus, le système comprenant:

    a) des moyens pour déterminer la valeur d'au moins un paramètre représentatif ($\Delta$Pi, $\Delta$Pj...) de l'évolution de la masse ($\Delta$P) du patient inter dialytique, pour au moins deux séances (i,j...),
    b) des moyens pour déterminer la valeur (CPi, CPj, ...) d'au moins un paramètre représentatif de la conductivité plasmatique (CP) du patient pour au moins deux séances (i,j),
    c) des moyens pour déterminer la valeur (TMi, TMj) d'un paramètre représentatif du transfert de masse ionique du traitement pour au moins deux séances (i,j),

    d) des moyens programmés pour déterminer l'appartenance à un groupe d'hypertendus du patient en fonction de l'évolution sur plusieurs séances d'au moins un des trois ensembles suivants de valeurs:

        - un premier ensemble d'au moins deux valeurs ($\Delta$Pi, $\Delta$Pj) déterminées de l'évolution de la masse ($\Delta$P) du patient inter dialytique,
        - un deuxième ensemble d'au moins deux valeurs (CPi, CPj...) déterminées représentatives de la conductivité plasmatique et;
        - un troisième ensemble d'au moins deux valeurs (TMi, TMj) déterminées représentatives du transfert de masse ionique.

2. Système selon la revendication précédente où les groupes d'hypertension sont divisés en un premier groupe de patients hypertendus, un deuxième groupe de patients hypertendus, et un troisième groupe de patients hypertendus.

3. Système selon une des revendications précédentes où les moyens programmés pour déterminer l'appartenance à un groupe d'hypertendus comportent des moyens programmés pour déterminer si le patient appartient au premier groupe.

4. Système selon la revendication précédente où les moyens programmés pour déterminer l'appartenance à un groupe d'hypertendus comportent des moyens programmés pour déterminer si si le patient appartient au deuxième groupe lorsque le patient n'appartient pas au premier groupe.

5. Système selon la revendication précédente où les moyens programmés pour déterminer l'appartenance à un groupe d'hypertendus comportent des moyens programmés pour déterminer si le patient appartient au troisième groupe lorsque le patient n'appartient pas au deuxième groupe.

6. Système selon une des revendications précédentes où la valeur représentative ($\Delta$Pi, APj...) de l'évolution de la masse ($\Delta$P) du patient inter dialytique, pour au moins deux séances (i,j...) est choisie parmi le groupe comprenant:

    - - la prise de poids du patient entre la fin d'une séance (i) et le début de la séance suivante (i+1),
    - la prise de masse du patient entre la fin d'une séance (i) et le début de la séance suivante (i+1),
    - un paramètre proportionnel à un des précités paramètres.

7. Système selon une des revendications précédentes où le paramètre représentatif de la conductivité plasmatique est égale ou fonction d'au moins un des paramètres suivants :

- la conductivité plasmatique du patient,
- la conductivité plasmatique prédialytique du patient c'est-à-dire la conductivité plasmatique avant la séance de traitement extracorporel de sang ou,
- un paramètre proportionnel à un des précités paramètres.

8.  Système selon une des revendications précédentes où le paramètre représentatif du transfert de masse ionique pour au moins deux séances (i,j), est égal ou fonction d'au moins un des paramètres suivants :

    - le transfert de masse ionique pendant la séance du patient,
    - le transfert de masse de sodium pendant la séance du patient,
    - un paramètre proportionnel à un des précités paramètres.

9.  Système selon une des revendications précédentes comportant des moyens pour déterminer la tension (mmHg) du patient.

10. Système selon une des deux revendications précédentes où le patient est initialement considéré hypertendu lorsque les valeurs représentatives de sa tension augmentent d'au moins 20mmHg sur au moins deux séances et/ou la valeur représentative de sa tension pour une séance est supérieure à 150mmHG.

11. Système selon la revendication précédente où les moyens programmés pour déterminer si le patient appartient au premier groupe d'hypertendus sont des moyens programmés pour déterminer si les valeurs représentatives de l'évolution de masse du patient inter dialytique ($\Delta P$) sur plusieurs séances (i,j) montrent une tendance de diminution, le patient étant considéré comme appartenant au premier groupe quand la tendance est à la diminution.

12. Système selon la revendication précédente où les moyens de détermination d'appartenance au premier groupe d'hypertendus considéreront l'appartenance au premier groupe lorsque l'évolution des valeurs représentatives de la masse est supérieure à un kilogramme.

13. Système selon une des revendications précédentes où les moyens programmés pour déterminer si le patient appartient au deuxième groupe d'hypertendus sont des moyens programmés pour déterminer si les valeurs représentatives de la conductivité plasmatique (CP) sur plusieurs séances (i,j) montrent une tendance à l'augmentation ou additionnellement une tendance à la diminution, le patient étant considéré comme appartenant au deuxième groupe quand la tendance est à l'augmentation, ou additionnellement à une diminution.

14. Système selon la revendication précédente où les moyens pour déterminer si le patient appartient au deuxième groupe d'hypertendus considéreront l'appartenance au deuxième groupe lorsque les valeurs représentatives de la conductivité plasmatique augmentent ou lorsque les valeurs représentatives de la conductivité plasmatique diminuent de moins 0,3mS/cm.

15. Système selon une des revendications 1 à 12 où les moyens programmés pour déterminer la non appartenance au deuxième groupe d'hypertendus sont des moyens programmés pour déterminer si les valeurs représentatives de la conductivité plasmatique (CP) sur plusieurs séances (i,j) montrent une tendance à la diminution.

16. Système selon la revendication précédente où les moyens programmés pour déterminer la non appartenance au deuxième groupe d'hypertendus considéreront la non appartenance au deuxième groupe lorsque les valeurs représentatives de la conductivité plasmatique diminuent d'au moins 0,3mS/cm.

17. Système selon une des revendications précédentes où les moyens programmés pour déterminer l'appartenance au troisième groupe d'hypertendus sont des moyens programmés pour déterminer si les valeurs représentatives du transfert de masse ionique (TM) sur plusieurs séances (i,j) montre une tendance de diminution, le troisième niveau étant considéré rempli quand la tendance est à la diminution.

18. Système selon la revendication précédente où les moyens de détermination de l'appartenance au troisième groupe d'hypertendus considéreront l'appartenance au troisième groupe lorsque les valeurs représentatives de la conductivité plasmatique diminuent d'au moins 200mmol/séance.

19. Système selon une des revendications précédentes comprenant des moyens programmés pour recommander ou contrôler la modification d'au moins un paramètre médical ou d'au moins un paramètre machine pour chaque groupe d'hypertendus.

20. Système selon la revendication précédente comportant des moyens programmés pour, lorsque l'appartenance au premier groupe d'hypertendus est considérée, proposer ou contrôler lors d'une future séance de diminuer la masse ou le poids du patient devant être atteinte en fin de séance, où les moyens commandés pour proposer ou contrôler ladite diminution de la masse patient sont commandés pour que la diminution de la valeur représentative de la masse soit d'au moins environ 0,5 kg, voire environ 1kg.

21. Système selon la revendication 19 comportant des moyens programmés pour, lorsque l'appartenance au deuxième groupe d'hypertendus est considérée, proposer la diminution de la quantité de sel injecté par le patient d'une séance à l'autre.

22. Système selon la revendication 19 comportant des moyens programmés pour, lorsque l'appartenance au troisième groupe d'hypertendus est considérée, proposer ou contrôler lors d'une future séance de diminuer la valeur représentative de la conductivité dialytique.

23. Système selon la revendication précédente où la diminution proposée ou contrôlée de la conductivité dialytique est d'au moins 0,2mmol.

24. Machine de traitement extracorporel de sang comprenant au moins:

- une unité de traitement de sang capable de mettre en oeuvre un traitement extracorporel sanguin par circulation de sang via un circuit extracorporel sanguin comprenant une ligne artérielle, une première chambre d'un filtre séparé par une membrane semi-perméable, une ligne veineuse et par circulation de dialysat dans une deuxième chambre du filtre,
- des moyens de mémorisation stockant au moins des valeurs d'au moins un paramètre représentatif (Pi, Pj...) de la masse (P) du patient, au moins des valeurs d'au moins un paramètre représentatif (CPi, CPj...) de la conductivité plasmatique du patient et des valeurs d'au moins un paramètre représentatif du transfert de masse ionique (TMi, TMj...) relatives à au moins un patient soumis à plusieurs séances (i,...j) de traitement extracorporel sanguin,
- un système de calcul et de commande selon une des revendications 1 à 28 pour la détermination de l'appartenance à un groupe d'hypertendus du patient dont les valeurs de paramètres d'au moins un paramètre représentatif (Pi, Pj...) de la masse (P) du patient, des valeurs d'au moins un paramètre représentatif (CPi, CPj...) de la conductivité plasmatique du patient et des valeurs d'au moins un paramètre représentatif du transfert de masse ionique (TMi, TMj...) sont stockées dans lesdits moyens de mémorisation.

**Patentansprüche**

1. Rechen- und Steuersystem zum Bestimmen der Zugehörigkeit eines Patienten, der einer Vielzahl von Sitzungen (i, j) einer extrakorporalen Blutbehandlung mittels Blutentnahme und -rückgabe durch den Gefäßzugang unterworfen werden soll, zu einer Gruppe aus einer Vielzahl von Gruppen von hypertonischen Patienten, wobei das System umfasst:

a) Mittel zum Bestimmen des Wertes von mindestens einem Parameter ($\Delta$Pi, $\Delta$Pj...), der den Verlauf der interdialytische Masse ($\Delta$P) des Patienten darstellt, über mindestens zwei Sitzungen (i, j...),
b) Mittel zum Bestimmen des Wertes (CPi, CPj, ...) von mindestens einem Parameter, der die Plasmaleitfähigkeit (CP) des Patienten darstellt, über mindestens zwei Sitzungen (i, j),
c) Mittel zum Bestimmen des Wertes (TMi, TMj) von einem Parameter, der den Ionenmassentransfer der Behandlung darstellt, über mindestens zwei Sitzungen (i, j),
d) programmierte Mittel zum Bestimmen der Zugehörigkeit eines Patienten zu einer Gruppe von Hypertonikern abhängig vom Verlauf über eine Vielzahl von Sitzungen mindestens eines der folgenden drei Sätze von Werten:

- ein erster Satz von mindestens zwei bestimmten Werten ($\Delta$Pi, $\Delta$Pj) des Verlaufs der interdialytischen Masse ($\Delta$P) des Patienten,
- ein zweiter Satz von mindestens zwei bestimmten Werten (CPi, CPj...), die die Plasmaleitfähigkeit darstellen; und
- ein dritter Satz von mindestens zwei bestimmten Werten (TMi, TMj), die den Ionenmassentransfer darstellen.

2. System nach dem vorhergehenden Anspruch, wobei die Hypertoniegruppen in einer ersten Gruppe von hypertonischen Patienten, einer zweiten Gruppe von hypertonischen Patienten und einer dritten Gruppe von hypertonischen Patienten unterteilt sind.

3. System nach einem der vorhergehenden Ansprüche, wobei die programmierten Mittel zum Bestimmen der Zugehörigkeit zu einer Gruppe von Hypertonikern programmierte Mittel zum Bestimmen, ob der Patient zur ersten Gruppe zugehört, umfassen.

4. System nach dem vorhergehenden Anspruch, wobei die programmierten Mittel zum Bestimmen der Zugehörigkeit zu einer Gruppe von Hypertonikern programmierte Mittel zum Bestimmen, ob der Patient zur zweiten Gruppe zugehört, wenn der Patient zur ersten Gruppe nicht zugehört, umfassen.

**5.** System nach dem vorhergehenden Anspruch, wobei die programmierten Mittel zum Bestimmen der Zugehörigkeit zu einer Gruppe von Hypertonikern programmierte Mittel zum Bestimmen, ob der Patient zur dritten Gruppe zugehört, wenn der Patient zur zweiten Gruppe nicht zugehört, umfassen.

**6.** System nach einem der vorhergehenden Ansprüche, wobei der Wert ($\Delta$Pi, $\Delta$Pj...), der den Verlauf der interdialytischen Masse ($\Delta$P) des Patienten über mindestens zwei Sitzungen (i, j...) darstellt, ausgewählt ist aus der Gruppe umfassend:

- die Gewichtszunahme des Patienten zwischen dem Ende einer Sitzung (i) und dem Anfang der folgenden Sitzung (i+1),
- die Massenzunahme des Patienten zwischen dem Ende einer Sitzung (i) und dem Anfang der folgenden Sitzung (i+1),
- einen Parameter proportional zu einem der vorgenannten Parameter.

**7.** System nach einem der vorhergehenden Ansprüche, wobei der Parameter, der die Plasmaleitfähigkeit darstellt, gleich ist wie oder abhängig ist von mindestens einem der folgenden Parameter:

- der Plasmaleitfähigkeit des Patienten,
- der prädialytischen Plasmaleitfähigkeit des Patienten, d.h. der Plasmaleitfähigkeit vor der Sitzung einer extrakorporalen Blutbehandlung, oder
- einem Parameter proportional zu einem der vorgenannten Parameter.

**8.** System nach einem der vorhergehenden Ansprüche, wobei der Parameter, der den Ionenmassentransfer über mindestens zwei Sitzungen (i, j) darstellt, gleich ist wie oder abhängig ist von mindestens einem der folgenden Parameter:

- dem Ionenmassentransfer während der Sitzung des Patienten,
- dem Natriummassentransfer während der Sitzung des Patienten,
- einem Parameter proportional zu einem der vorgenannten Parameter.

**9.** System nach einem der vorhergehenden Ansprüche, umfassend Mittel zum Bestimmen des Drucks (mmHg) des Patienten.

**10.** System nach einem der beiden vorhergehenden Ansprüche, wobei der Patient anfänglich als hypertonisch betrachtet wird, wenn die seinen Druck darstellenden Werte um mindestens 20 mmHg über mindestens zwei Sitzungen zunehmen und/oder wenn der seinen Druck darstellende Wert über eine Sitzung mehr als 150 mmHg beträgt.

**11.** System nach dem vorhergehenden Anspruch, wobei die programmierten Mittel zum Bestimmen, ob der Patient zur ersten Gruppe von Hypertonikern zugehört, programmierte Mittel sind zum Bestimmen, ob die Werte, die den Verlauf der interdialytischen Masse ($\Delta$P) des Patienten über eine Vielzahl von Sitzungen (i, j) darstellen, eine Tendenz zur Abnahme zeigen, wobei der Patient als zur ersten Gruppe zugehörend betrachtet wird, wenn die Tendenz zur Abnahme vorliegt.

**12.** System nach dem vorhergehenden Anspruch, wobei die Mittel zum Bestimmen der Zugehörigkeit zur ersten Gruppe von Hypertonikern die Zugehörigkeit zur ersten Gruppe betrachten werden, wenn der Verlauf der die Masse darstellenden Werte mehr als 1 Kilogramm beträgt.

**13.** System nach einem der vorhergehenden Ansprüche, wobei die programmierten Mittel zum Bestimmen, ob der Patient zur zweiten Gruppe von Hypertonikern zugehört, programmierte Mittel sind zum Bestimmen, ob die Werte, die die Plasmaleitfähigkeit (CP) über eine Vielzahl von Sitzungen (i, j) darstellen, eine Tendenz zur Zunahme oder zusätzlich zur Abnahme zeigen, wobei der Patient als zur zweiten Gruppe zugehörend betrachtet wird, wenn die Tendenz zur Zunahme oder zusätzlich zur Abnahme vorliegt.

**14.** System nach dem vorhergehenden Anspruch, wobei die Mittel zum Bestimmen, ob der Patient zur zweiten Gruppe von Hypertonikern zugehört, die Zugehörigkeit zur zweiten Gruppe betrachten werden, wenn die die Plasmaleitfähigkeit darstellenden Werte zunehmen oder wenn die die Plasmaleitfähigkeit darstellenden Werte um weniger als 0,3 mS/cm abnehmen.

**15.** System nach einem der Ansprüche 1 bis 12, wobei die programmierten Mittel zum Bestimmen der Nicht-Zugehörigkeit zur zweiten Gruppe von Hypertonikern programmierten Mittel sind zum Bestimmen, ob die die Plasmaleitfähigkeit (CP) über eine Vielzahl von Sitzungen (i, j) darstellenden Werte eine Tendenz zur Abnahme zeigen.

**16.** System nach dem vorhergehenden Anspruch, wobei die programmierten Mittel zum Bestimmen der Nicht-Zugehörigkeit zur zweiten Gruppe von Hypertonikern die Nicht-Zugehörigkeit zur zweiten Gruppe betrachten werden, wenn die die Plasmaleitfähigkeit darstellenden Werte um mindestens 0,3 mS/cm abnehmen.

**17.** System nach einem der vorhergehenden Ansprüche, wobei die programmierten Mittel zum Bestimmen der Zugehörigkeit zur dritten Gruppe von Hypertonikern programmierte Mittel sind zum Bestimmen, ob die den Ionenmassentransfer (TM) über eine Vielzahl von Sitzungen (i, j) darstellenden Werte eine Tendenz zur Abnahme zeigen, wobei das dritte Niveau als erfüllt betrachtet wird, wenn die Tendenz zur Abnahme vorliegt.

18. System nach dem vorhergehenden Anspruch, wobei die Mittel zum Bestimmen der Zugehörigkeit zur dritten Gruppe von Hypertonikern die Zugehörigkeit zur dritten Gruppe betrachten werden, wenn die die Plasmaleitfähigkeit darstellenden Werte um mindestens ca. 200 mMol/Sitzung abnehmen.

19. System nach einem der vorhergehenden Ansprüche, umfassend programmierten Mittel zum Empfehlen oder Steuern der Änderung von mindestens einem medizinischen Parameter oder von mindestens einem Maschinenparameter für jede Gruppe von Hypertonikern.

20. System nach einem der vorhergehenden Ansprüche, umfassend programmierte Mittel zum, wenn die Zugehörigkeit zur ersten Gruppe von Hypertonikern betrachtet wird, Vorschlagen oder Steuern während einer zukünftigen Sitzung, die am Ende der Sitzung zu erreichende Masse oder das zu erreichende Gewicht des Patienten abzunehmen, wobei die programmierten Mittel zum Vorschlagen oder Steuern der Abnahme der Masse der Patienten gesteuert werden, damit die Abnahme des die Masse darstellenden Wertes mindestens ca. 0,5 Kg bzw. ca. 1 Kg beträgt.

21. System nach Anspruch 19, umfassend programmierte Mittel zum, wenn die Zugehörigkeit zur zweiten Gruppe von Hypertonikern betrachtet wird, Vorschlagen der Abnahme der Salzmenge, die von einer zur anderen Sitzung in den Patienten injiziert wird.

22. System nach Anspruch 19, umfassend programmierte Mittel zum, wenn die Zugehörigkeit zur dritten Gruppe von Hypertonikern betrachtet wird, Vorschlagen oder Steuern während einer zukünftigen Sitzung, den die dialytische Leitfähigkeit darstellenden Wert abzunehmen.

23. System nach dem vorhergehenden Anspruch, wobei die vorgeschlagene oder gesteuerte Abnahme der dialytischen Leitfähigkeit mindestens 0,2 mMol beträgt.

24. Maschine zur extrakorporalen Blutbehandlung, umfassend mindestens:

- eine Blutbehandlungseinheit, die eine extrakorporale Blutbehandlung durchführen kann, mittels Blutzirkulation durch einen extrakorporalen Blutkreislauf umfassend eine arterielle Leitung, eine erste Kammer eines Filters, der durch eine semipermeable Membran getrennt ist, eine venöse Leitung, und mittels Dialysatzirkulation in einer zweiten Kammer des Filters,
- Speichermittel, die mindestens Werte von mindestens einem Parameter (Pi, Pj...), der die Masse (P) des Patienten darstellt, mindestens Werte von mindestens einem Parameter (CPi, CPj...), der die Plasmaleitfähigkeit des Patienten darstellt, und Werte von mindestens einem Parameter, der den Ionenmassentransfer (TMi, TMj...) darstellt, für mindestens einen Patienten, der einer Vielzahl von Sitzungen (i, ...j) einer extrakorporalen Blutbehandlung unterworfen wird,
- ein Rechen- und Steuersystem nach einem der Ansprüche 1 bis 28, zum Bestimmen der Zugehörigkeit zu einer Gruppe von Hypertonikern des Patienten, dessen Parameterwerte von mindestens einem Parameter (Pi, Pj...), der die Masse (P) des Patienten darstellt, Werte von mindestens einem Parameter (CPi, CPj...), der die Plasmaleitfähigkeit des Patienten darstellt, und Werte von mindestens einem Parameter, der den Ionenmassentransfer (TMi, TMj...) darstellt, in den Speichermitteln gespeichert sind.

## Claims

1. A calculation and control system for determining whether a patient intended to follow several sessions (i, j) of extracorporeal blood treatment by extraction and return of blood via the vascular access, falls within a group among several groups of hypertension-affected patients, the system comprising:

    a) means for determining the value of at least one parameter (ΔPi, ΔPj...) representing the evolution of the interdialytic mass (ΔP) of the patient, for at least two sessions (i, j...),
    b) means for determining the value (CPi, CPj, ...) of at least one parameter representing the plasmatic conductivity (CP) of the patient for at least two sessions (i, j),
    c) means for determining the value (TMi, TMj) of a parameter representing the ionic mass transfer of the treatment for at least two sessions (i, j),
    d) programmed means for determining whether the patient falls within a group of hypertension-affected patients as a function of the evolution over several sessions of at least one of the three following sets of values:

- a first set of at least two determined values ($\Delta$Pi, $\Delta$Pj) of the evolution of the interdialytic mass ($\Delta$P) of the patient,
- a second set of at least two determined values (CPi, CPj...) representing the plasmatic conductivity; and
- a third set of at least two determined values (TMi, TMj) representing the ionic mass transfer.

2. The system according to the preceding claim, wherein the hypertension groups are divided into a first group of hypertension-affected patients, a second group of hypertension-affected patients and a third group of hypertension-affected patients.

3. The system according to one of the preceding claims, wherein the programmed means for determining the belonging to a group of hypertension-affected patients comprise programmed means for determining whether the patient falls within the first group.

4. The system according to the preceding claim, wherein the programmed means for determining the belonging to a group of hypertension-affected patients comprise programmed means for determining whether the patient falls within the second group when the patient does not fall within the first group.

5. The system according to the preceding claim, wherein the programmed means for determining the belonging to a group of hypertension-affected patients comprise programmed means for determining whether the patient falls within the third group when the patient does not fall within the second group.

6. The system according to one of the preceding claims, wherein the value ($\Delta$Pi, $\Delta$Pj...) representing the evolution of the interdialytic mass ($\Delta$P) of the patient for at least two sessions (i, j...), is chosen from the group comprising:

   - the weight increase of the patient between the end of one session (i) and the beginning of the following session (i+1),
   - the mass increase of the patient between the end of one session (i) and the beginning of the following session (i+1),
   - a parameter proportional to one of the aforesaid parameters.

7. The system according to one of the preceding claims, wherein the parameter representing the plasmatic conductivity is the same as or function of at least one of the following parameters:

   - the plasmatic conductivity of the patient,

   - the predialytic plasmatic conductivity of the patient, i.e. the plasmatic conductivity before the session of extracorporeal blood treatment, or
   - a parameter proportional to one of the aforesaid parameters.

8. The system according to one of the preceding claims, wherein the parameter representing the ionic mass transfer for at least two sessions (i, j) is the same as or function of at least one of the following parameters:

   - the ionic mass transfer during the patient's session,
   - the sodium mass transfer during the patient's session,
   - a parameter proportional the one of the aforesaid parameters.

9. The system according to one of the preceding claims, comprising means for determining the patient's pressure (mmHg).

10. The system according to one of the two preceding claims, wherein the patient is initially regarded as hypertension-affected when the values representing his/her pressure increase of at least about 20 mmHg over at least two sessions and/or the value representing his/her pressure for one session is above 150 mmHg.

11. The system according to the preceding claim, wherein the programmed means for determining whether the patient falls within the first group of hypertension-affected patients are programmed means for determining whether the values representing the evolution of the interdialytic mass ($\Delta$P) of the patient over several sessions (i, j) show a tendency to decrease, the patient being regarded as falling within the first group when the tendency is to decrease.

12. The system according to the preceding claim, wherein the means for determining the belonging to the first group of hypertension-affected patients will consider the belonging to the first group when the evolution of the values representing the mass is above about one kilogram.

13. The system according to one of the preceding claims, wherein the programmed means for determining whether the patient falls within the second group of hypertension-affected patients are programmed means for determining whether the values representing the plasmatic conductivity (CP) over several sessions (i, j) show a tendency to increase or furthermore a tendency to decrease, the patient being considered as falling within the second group when the tendency is to increase or furthermore to decrease.

14. The system according to the preceding claim, wherein the means for determining whether the patient belongs to the second group of hypertension-affected patients will consider the belonging to the second group when the values representing the plasmatic conductivity increase or when the values representing the plasmatic conductivity decrease of less than about 0.3 mS/cm.

15. The system according to one of the claims 1 to 12, wherein the programmed means for determining the non-belonging to the second group of hypertension-affected patients are programmed means for determining whether the values representing the plasmatic conductivity (CP) over several sessions (i, j) show a tendency to decrease.

16. The system according to the preceding claim, wherein the programmed means for determining the non-belonging to the second group of hypertension-affected patients will consider the non-belonging to the second group when the values representing the plasmatic conductivity decrease of at least 0.3 mS/cm.

17. The system according to one of the preceding claims, wherein the programmed means for determining the belonging to the third group of hypertension-affected patients are programmed means for determining whether the values representing the ionic mass transfer (TM) over several sessions (i, j) show a tendency to decrease, the third level being regarded as fulfilled when the tendency is to decrease.

18. The system according to the preceding claim, wherein the means for determining the belonging to the third group of hypertension-affected patients will consider the belonging to the third group when the values representing the plasmatic conductivity decrease of at least about 200 mmol/session.

19. The system according to one of the preceding claims, comprising programmed means for urging or monitoring the change of at least one medical parameter or of at least one machine parameter for each group of hypertension-affected patients.

20. The system according to the preceding claim, comprising programmed means for, when the belonging to the first group of hypertension-affected patients is taken into account, suggesting or monitoring during a future session the decrease in the patient's mass or weight to be reached at the end of the session, wherein the programmed means for suggesting or monitoring said decrease in the patient's mass are controlled so that the decrease in the value representing the mass is of at least about 0.5 kg, or about 1 kg.

21. The system according to claim 19, comprising programmed means for, when the belonging to the second group of hypertension-affected patients is taken into account, suggesting the decrease in the amount of salt taken by the patient from one session to the other.

22. The system according to claim 19, comprising programmed means for, when the belonging to third group of hypertension-affected patients is taken into account, suggesting or monitoring during a future session the decrease in the value representing the dialytic conductivity.

23. The system according to the preceding claim, wherein the suggested or monitored decrease in dialytic conductivity is of at least 0.2 mmol.

24. An extracorporeal blood treatment machine comprising at least:

- a blood treatment unit capable of implementing an extracorporeal blood treatment by blood circulation via an extracorporeal blood circuit comprising an arterial line, a first chamber of a filter separated by a semi-permeable membrane, a venous line, and by dialysate circulation in a second chamber of the filter,
- storage means storing at least values of at least one parameter (Pi, Pj...) representing the patient's mass (P), at least values of at least one parameter (CPi, CPj...) representing the patient's plasmatic conductivity, and values of at least one parameter representing the ionic mass transfer (TMi, TMj...) relating to at least one patient undergoing several sessions (i, ...j) of extracorporeal blood treatment,
- a calculation and control system according to one of the claims 1 to 28 for determining whether the patient whose parametric values of at least one parameter (Pi, Pj...) representing the patient's mass (P), whose values of at least one parameter (CPi, CPj...) representing the patient's plasmatic conductivity and whose values of at least one parameter representing the ionic mass transfer (TMi, TMj...) are stored in said storage means, falls within a group of hypertension-affected patients.

Fig 1

Fig 2

Fig 3

Fig 4

Fig 5

Fig 6

Fig 7

Fig 8

Fig 9

Fig 10

Fig 11

Fig 12

Fig 13

Fig 14

Fig 15

Fig 16

Fig 17

Fig 18

Fig 19

## RÉFÉRENCES CITÉES DANS LA DESCRIPTION

**Documents brevets cités dans la description**

- US 6110384 A
- EP 0547025 A
- FR 0700298
- EP 0920877 A

**Littérature non-brevet citée dans la description**

- **WILLIMA L. EINRICH.** *principles and practice of dialysis, second édition*
- **C.CHAZOT ; B.CHARRA.** *néphrologie et thérapeutique - hypertension et dialyse,* Octobre 2007, vol. 3 (3
- **WENDY PURCELL ; ELISABETH MANIAS ; ALLISON WILLIAMS ; ROWAN WALKER.** *nephrology nursing,* Novembre 2004, vol. 31 (6
- **GOTCH FA ; SARGENT SA.** A mechanistic analysis of the National Coopérative Dialysis Study (NCDS). *Kidney int,* 1985, vol. 28, 526-34